# EUROPEAN PATENT APPLICATION

(11) **EP 4 012 018 A1**
(43) Date of publication of application: **15.06.2022**
(21) Application number: 20207123.9
(22) Date of filing: 12.11.2020
(51) Int. Cl.: C12N 1/14

(54) **PROCESS FOR THE PRODUCTION OF A TECHNICAL ENZYME COMPOSITION WITH LOW VISCOSITY PRODUCED BY A FILAMENTOUS FUNGUS**

(71) Applicant: Clariant Produkte (Deutschland) GmbH, 65929 Frankfurt am Main (DE)
(72) Inventor: GAMAUF, Christian, 81241 München (DE); CLAREN, Jörg, 82131 Stockdorf (DE)
(74) Representative: Graser, Konstanze

(57) **Abstract**

The present invention relates to a process for the production of a technical enzyme composition with low viscosity produced by a genetically modified filamentous fungus, a genetically modified filamentous fungus suitable for production of the technical enzyme composition, the use of such a genetically modified filamentous fungus for the production of the technical enzyme composition with low viscosity and a technical enzyme composition with low viscosity produced by such a process.

SEQUENCE LISTING

<110> Clariant Produkte (Deutschland) GmbH

<120> PROCESS FOR THE PRODUCTION OF A TECHNICAL ENZYME COMPOSITION WITH LOW VISCOSITY PRODUCED BY A FILAMENTOUS FUNGUS

<130> 2020DE620-EP-EPA

<160> 4

<170> BiSSAP 1.3.6

<210> 1
<211> 5163
<212> DNA
<213> Trichoderma reesei QM6a

<220>
<223> native gene

<400> 1
atggtttctg gcgactacgc cttcaacccc gatcaacacg gcgcatatgc cgaaccgtac 60

caacagccgg acgacggccg gactaggacg ctgcttgaca accaagcctt cttttctgac 120

ttcgcgggcc agcagcacta cgaacagaac cagatgggcg actatggtgg ccctagatac 180

tccggcgatg ccttctctcc gacagcagcc atggctcctc cgatgctcac tgccaacgac 240

atgcctccac ccgagatatt ggagtaccag gctccgctcg agccaagaga ggtccccttt 300

gccattcagg atccccacga caacaacacg gccatgtctt cgttcgacaa catggctgcg 360

gtactccgtc accgtgcccg caccactgcc aaaagacctg catattgggt cctggacagc 420

aagggcaagg aggtggcatc cattacatgg gacaagctgg cgtcaagagc ggagaaagtt 480

gcacaagtca tccgagacaa aagtcctctt taccgtggcg atcgagttgc cttgatctat 540

cgtgatagcg aaatcattga cttcgccatt gccttgctgg gttgcttcat tgctggagtt 600

gtggctgtcc cgatcaatga cttgcaagac taccagcgcc tcaactatat tctcacctcg 660

actcaggcgc atctggctct tactaccgaa aacaacctca agaccttcca gagagacatt 720

actgcgcaga agctcacgtg gcctaaaggg gtcgagtggt ggaagaccaa cgagttcggc 780

ggttaccatc cgaagaagaa ggaagacgca cctccgttaa ctgttcccga cctggcctat 840

attgagtttt cgcgagcacc aaccggcgac ttgaggggcg ttgttctcag ccacaggaca 900

atcatgcacc agatggcctg cctcagtgcc ataatctcta ccgttcccac caacggcccc 960

ggcgatacct tcaactcgac gttgcgggac aagaacggaa agctcatcgg cggcggagcc 1020

agcagcgaga tattgctctc ctatctggat ccccgacagg gcgtgggcat gattctcggc 1080

gttttgctga ccgtttacgg cggccacact accgtctggt tcgatcacaa agccgtcgag 1140

tcgcctggct tatacgcgca tctgattacc aagtacagag cgacgattat gattgcggat 1200

taccccgggt tgaagcgagc tgcctacaac taccagcaag accccatgac gacacgaaac 1260

ttcaaaaagg ggatggaacc caacttccaa gcggtgaagc tgtgcttgat tgataccctg 1320

accattgata gcgagttcca tgaagttctg gccgatagat ggctgcggcc cctgcgaaat 1380

ccgcgagcgc gcgaggtcgt ggcgccgatg ctctgcctcc ccgagcatgg cggcatgatc 1440

attagcgttc gagactggct cggcggtgaa gaacgactgg gagttccgct gaaactggac 1500

gagtctgaca gggagtcgga tgacgagaaa gaagaggaag agaagccggc cccgtcaaac 1560

ggatttggta gcttgcttgg tggtggagca gcgacaacca aggagcagga cgagaagatt 1620

gagttgggcg aggttatcct tgaccgagag gctctcaaga ccaacgaggt tgtcgtcttg 1680

gctcatggca acgaagctag gaagaagacg tcgctggagc ccaccatggt ccgggtcggc 1740

gcctttggat accctatccc agatgccacg cttgctgttg tggaccctga gactggcctc 1800

ctggcagcgc cgcacacgat tggcgagatc tgggttgact ctccgtctct ctctggaggc 1860

ttctgggcgc agccaaagaa caccgagctc atcttccacg cgcgtccgta caagttcgag 1920

cctggcgagc cgacgccaac tgccgtggag ccggaattcc tgcgaaccgg cctgcttggc 1980

acagtcatcg agggcaagat ctatgtgcta ggattgtacg aggatcggat acgacaaaag 2040

gtcgaatggg ttgagcacgg ccacgcgggt atcgccgagt atcgctactt ctttgttcag 2100

cacattgtgg tgagcatcgt caagaatgtc cccaagatcc acgactgctc tgcctttgac 2160

gtctttgtca atgacgagca cttgcctgtc gtggtcctcg agtctgccgc agcatcaacg 2220

gcgcctctca cttcgggcgg cccccctgtc cagcctgaca cggttctgtt agactcgctg 2280

gcggagaaat gcatggaggt gctcatgcag gagcaccatc ttcgggttta ctgcgttatg 2340

atcacagccc cgaacgcact gccgcgagtg atcaagaacg gaagacggga aatagggaac 2400

atgctctgcc ggcgcgagtt tgaccttggc aacctcccat gcgtgcatgt caaatttggc 2460

gtcgaacatg cggttctcaa cctcccgatt ggcgttgacc ccattggtgg tatctggtca 2520

ccaatcgcct cggactcgag aatcaatatc ctggctcccg ccgataagca gtattctgga 2580

atcgaccgca gagaggttgt tatggacgac cggacgtcta caccgctcaa caatttcaag 2640

accatcaccg atctgatcca gtggcgtgtt gctcgccagc cagaggagct cgcttattgt 2700

accattgacg gcaggggcag agaggcaagg gggattccgt ggaagaagtt tgactccaag 2760

gtggcggctg tggccatgta tctgaagaac aaagtcaagg tgcggccggg cgaccacctg 2820

gtcctcatgt acacccactc cgaggagttt gtctttgccg tccacgcggg aatcaacctt 2880

ggcgcagtca ttattcccat ggcgccgctt gaccagaacc ggctcaacga agatgtccct 2940

gctttcctgc acctgatcgc tgactacaag gttaaggcgg tcctggtcaa ccaggaagtg 3000

gaccatttgc tgaagctcaa gatcgtgtcg agccacatca aacagtccgc acagatcctg 3060

aagatctcga tgccgaatac ctacaacact tcgaagccac ctaagcagaa caacggtctt 3120

cgcgagcttg ggctgacgat agatcccgcc tggatcaggc ctggataccc cgtcctcatc 3180

tggacatact ggacgccgga ccaacggaga atcgccgtcc agctggggca tgataccatc 3240

ctgggcatgt gcaaagtgca gaaggagact tgtcagatga cgagcttcca gcccgttctc 3300

ggttgcgtaa gaagcacaac gggacttggt ttcgtgcaca cgtgcctgat gggcatctac 3360

gttggcaccg ccacctacct gctgtctcct gtcgagttcg cccaaaatcc catctctctc 3420

tttgttacgt tgtcgaggta caagatcaag gacacctatg caacgccgca gatgcttgac 3480

catgccatgt cgtcgatgca ggccaagggc tttacaatgc acgaactgaa gaatatgatg 3540

attactgcag agggccggcc gcgggtagat gtattccaga aggtacggat gcattttgcg 3600

agcgccgggc tggataggac ggccatcaac acggtctact cgcatgtgct caacccgatg 3660

attgcttcga ggtcttacat gtgcatcgag cctattgagc tctggctcga caccaaggct 3720

cttcgacgcg gcctcgtcgt cccggtcgat cacgattcaa acccgcaagc tcttctcctg 3780

caggattccg gcatggtgcc ggtgtctacc cagattgcca ttgtcaaccc cgagagccgc 3840

gcgcattgct acgatggaga atatggcgag atctgggtcg actccgaggc gtgcgtaaag 3900

gccttttacg gctccaagga agcgtttgac gtggagcgct tcgacggccg gacggtcgac 3960

ggcgacccca acgtgcgata cgtgcgaact ggtgacttgg gctttttgta taatgtcaac 4020

cggcctatcg ggcccaacgg cgccctggtg gagatgcaag tcttgtttgt gctcggtagc 4080

atcggcgaga cttttgaaat taacggtttg agtcacttcc ccatggatat tgagctgtcg 4140

gtggaacgct gccaccgcaa cattgtaccc aacggctggt aagtacaggg ccaactcttc 4200

tgtgagatgc tacttgacta atagttggtg atgtgcagtg ctgtattcca agctggtggc 4260

ttggtcgtgg tcctggtaga ggtgagccgc aagtcttacc tcgcctccat ggtgccagtc 4320

attgtcaacg ccatcttgag cgagcatcag atcgttgccg atattgtggc ctttgtcaac 4380

aagggcgact tcccacgctc tcgcctggga gagaagcagc gaggaaagat cctcgccgga 4440

tgggttacgc gcaagatgcg caccatggcc cagttcgcca tcagagatct cgacgccagc 4500

atgctcgagc cgggtgaggg tccggatgcc aataggacct ctacgggtag cctccgtagc 4560

ctgggcgccg ccgtcccgcc aaacttcaag atggttggac aggcgccgca gatactggaa 4620

caggaggaat ttacgcagca gatggatcac atggcccatt cggagccggt caggcatgcc 4680

atggccgctc cagaggagca gcaggcgccg gcggcctatt acgctggcgg ccaggaggct 4740

gctttcatgc agggctataa tcagcagccg ccaccaccac caccaagcag ccaggaggga 4800

taccagtacg agcaattcga gccagtgcaa gcacagcaac agtaccagcc gcagtcacag 4860

catcagtttg aaccatctca agcctttgag ccagcgcagc aatacgagcc agcgcaacag 4920

cacgagcagg agccaaggcc gatggactct cgaggacaag atgcgccgtc cattgttgag 4980

ccagagacct cagcctccgt gcctgatacg ccgccgccga gaaacaggtt gagtcaaggg 5040

ccgccccaga tccggctccc gggcgttgac ggacgggaag ggctcgactt ctggggaggc 5100

aacgacgaga cggactggac ggccgatgcc atgatgcaca tgaatctcac tggcccacgg 5160

taa 5163

<210> 2
<211> 2526
<212> DNA
<213> Artificial Sequence

<220>
<223> Hygromycin B resistance marker

<400> 2
tgcaaggcga ttaagttggg taacgccagg gttttcccag tcacgacgtt gtaaaacgac 60

ggccagtgag cgcgacgtaa tacgactcac tatagggcga attggcggaa ggccgtcaag 120

gcctaggcgc gccatgagct cgttaacaag acacagccct ataacttcgt ataatgtatg 180

ctatacgaag ttatataacg gtgagactag cggccggtcc ccttatccca gctgttccac 240

gttggcctgc ccctcagtta gcgctcaact caatgcccct cactggcgag gcgagggcaa 300

ggatggaggg gcagcatcgc ctgagttgga gcaaagcggc ccggccgcca tgggagcagc 360

gaaccaacgg agggatgccg tgctttgtcg tggctgctgt ggccaatccg ggcccttggt 420

tggctcacag agcgttgctg tgagaccatg agctattatt gctaggtaca gtatagagag 480

aggagagaga gagagagaga gagagagggg aaaaaaggtg aggttgaagt gagaaaaaaa 540

aaaaaaaaaa aaaatccaac cactgacggc tgccggctct gccacccccc tccctccacc 600

ccagaccacc tgcacactca gcgcgcagca tcacctaatc ttggctcgcc ttcccgcagc 660

tcaggttgtt ttttttttct ctctccctcg tcgaagccgc ccttgttccc ttatttattt 720

ccctctccat ccttgtctgc ctttggtcca tctgcccctt tgtctgcatc tcttttgcac 780

gcatcgcctt atcgtcgtct cttttttcac tcacgggagc ttgacgaaga cctgactcgt 840

gagcctcacc tgctgatttc tctccccccc tcccgaccgg cttgactttt gtttctcctc 900

cagtacctta tcgcgaagcc ggaagaacct cttaacctct agatgaaaaa gcctgaactc 960

accgccacgt ctgtcgagaa gttcctgatc gaaaagttcg acagcgtctc cgacctgatg 1020

cagctctcgg agggcgaaga atctcgtgct ttcagcttcg atgtaggagg gcgtggatat 1080

gtcctgcggg taaatagctg cgccgatggt ttctacaaag atcgttatgt ttatcggcac 1140

tttgcatcgg ccgcgctccc gattccggaa gtgcttgaca ttggggaatt cagcgagagc 1200

ctgacctatt gcatctcccg ccgtgcacag ggtgtcacgt tgcaagacct gcctgaaacc 1260

gaactgcccg ctgttctgca gccggtcgcg gaggccatgg atgcgatcgc tgcggccgat 1320

ctcagccaga cgagcgggtt cggcccattc ggaccgcaag gaatcggtca atacactaca 1380

tggcgtgatt tcatatgcgc gattgctgat ccccatgtgt atcactggca aactgtgatg 1440

gacgacaccg tcagtgcgtc cgtcgcgcag gctctcgatg agctgatgct ttgggccgag 1500

gactgccccg aagtccggca cctcgtgcac gcggatttcg gctccaacaa tgtcctgacg 1560

gacaatggcc gcataacagc ggtcattgac tggagcgagg cgatgttcgg ggattcccaa 1620

tacgaggtcg ccaacatctt cttctggagg ccgtggttgg cttgtatgga gcagcagacg 1680

cgctacttcg agcggaggca cccggagctt gcaggatcgc cgcggctccg ggcgtatatg 1740

ctccgcattg gtcttgacca actctatcag agcttggttg acggcaattt cgatgatgca 1800

gcttgggcgc agggtcgatg cgacgcaatc gtccgatccg gagccgggac tgtcgggcgt 1860

acacaaatcg cccgcagaag cgcggccgtc tggaccgatg gctgtgtaga agtactcgcc 1920

gatagtggaa accgacgccc cagcactcgt ccgagggcaa aggaatagat gcatggcttt 1980

cgtgaccggg cttcaaacaa tgatgtgcga tggtgtggtt cccggttggc ggagtctttg 2040

tctactttgg ttgtctgtcg caggtcggta gaccgcaaat gagcaactga tggattgttg 2100

ccagcgatac tataattcac atggatggtc tttgtcgatc agtagctagt gagagagaga 2160

gaacatctat ccacaatgtc gagtgtctat tagacatact ccgagaataa agtcaactgt 2220

gtctgtgatc taaagatcga ttcggcagtc gagtagcgta taacaactcc gagtaccagc 2280

gaaagcacgt cgtgacagga gcagggcttt gccaactgcg caaccttgct tgaatgagga 2340

tacacggggt gcaacatggc tgtactgatc catcgcaacc aaaatttctg tttatagatc 2400

aagctggtag attccaatta ctccacctct tgcgcttctc catgacatgt aagtgcacgt 2460

ggaaaccata cccaatataa cttcgtataa tgtatgctat acgaagttat agggctcttg 2520

tctgtt 2526

<210> 3
<211> 1846
<212> DNA
<213> Artificial Sequence

<220>
<223> LIC reception vector

<400> 3
tttatcaggg ttattgtctc atgagcggat acatatttga atgtatttag aaaaataaac 60

aaataggggt tccgcgcaca tttccccgaa aagtgccacc tgactaaacc agacagacag 120

ctgtctctcc tctctaacat gtgagcaaaa ggccagcaaa aggccaggaa ccgtaaaaag 180

gccgcgttgc tggcgttttt ccataggctc cgcccccctg acgagcatca caaaaatcga 240

cgctcaagtc agaggtggcg aaacccgaca ggactataaa gataccaggc gtttccccct 300

ggaagctccc tcgtgcgctc tcctgttccg accctgccgc ttaccggata cctgtccgcc 360

tttctccctt cgggaagcgt ggcgctttct catagctcac gctgtaggta tctcagttcg 420

gtgtaggtcg ttcgctccaa gctgggctgt gtgcacgaac cccccgttca gcccgaccgc 480

tgcgccttat ccggtaacta tcgtcttgag tccaacccgg taagacacga cttatcgcca 540

ctggcagcag ccactggtaa caggattagc agagcgaggt atgtaggcgg tgctacagag 600

ttcttgaagt ggtggcctaa ctacggctac actagaagga cagtatttgg tatctgcgct 660

ctgctgaagc cagttacctt cggaaaaaga gttggtagct cttgatccgg caaacaaacc 720

accgctggta gcggtggttt ttttgtttgc aagcagcaga ttacgcgcag aaaaaaagga 780

tctcaagaag atcctttgat cttttctacg gggtctgacg ctcagtggaa cgaaaactca 840

cgttaaggga ttttggtcat gagattatca aaaaggatct tcacctagat ccttttaaat 900

taaaaatgaa gttttaaatc aatctaaagt atatatgagt aaacttggtc tgacagttac 960

caatgcttaa tcagtgaggc acctatctca gcgatctgtc tatttcgttc atccatagtt 1020

gcctgactcc ccgtcgtgta gataactacg atacgggagg gcttaccatc tggccccagt 1080

gctgcaatga taccgcgaga cccacgctca ccggctccag atttatcagc aataaaccag 1140

ccagccggaa gggccgagcg cagaagtggt cctgcaactt tatccgcctc catccagtct 1200

attaattgtt gccgggaagc tagagtaagt agttcgccag ttaatagttt gcgcaacgtt 1260

gttgccattg ctacaggcat cgtggtgtca cgctcgtcgt ttggtatggc ttcattcagc 1320

tccggttccc aacgatcaag gcgagttaca tgatccccca tgttgtgcaa aaaagcggtt 1380

agctccttcg gtcctccgat cgttgtcaga agtaagttgg ccgcagtgtt atcactcatg 1440

gttatggcag cactgcataa ttctcttact gtcatgccat ccgtaagatg cttttctgtg 1500

actggtgagt actcaaccaa gtcattctga gaatagtgta tgcggcgacc gagttgctct 1560

tgcccggcgt caatacggga taataccgcg ccacatagca gaactttaaa agtgctcatc 1620

attggaaaac gttcttcggg gcgaaaactc tcaaggatct taccgctgtt gagatccagt 1680

tcgatgtaac ccactcgtgc acccaactga tcttcagcat cttttacttt caccagcgtt 1740

tctgggtgag caaaaacagg aaggcaaaat gccgcaaaaa agggaataag ggcgacacgg 1800

aaatgttgaa tactcatact cttccttttt caatattatt gaagca 1846

<210> 4
<211> 4436
<212> DNA
<213> Artificial Sequence

<220>
<223> pSEQ1flank5

<400> 4
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtggccgct acagggcgct cccattcgcc attcaggctg cgcaactgtt 180
   gggaagggcg tttcggtgcg ggcctcttcg ctattacgcc agctggcgaa agggggatgt 240
   gctgcaaggc gattaagttg ggtaacgcca gggttttccc agtcacgacg ttgtaaaacg 300
   acggccagtg agcgcgacgt aatacgactc actatagggc gaattggcgg aaggccgtca 360
   aggccgcatg actgaataac ttcgggcggc ccccctgtcc agcctgacac ggttctgtta 420
   gactcgctgg cggagaaatg catggaggtg ctcatgcagg agcaccatct tcgggtttac 480
   tgcgttatga tcacagcccc gaacgcactg ccgcgagtga tcaagaacgg aagacgggaa 540
   atagggaaca tgctctgccg gcgcgagttt gaccttggca acctcccatg cgtgcatgtc 600
   aaatttggcg tcgaacatgc ggttctcaac ctcccgattg gcgttgaccc cattggtggt 660
   atctggtcac caatcgcctc ggactcgaga atcaatatcc tggctcccgc cgataagcag 720
   tattctggaa tcgaccgcag agaggttgtt atggacgacc ggacgtctac accgctcaac 780
   aatttcaaga ccatcaccga tctgatccag tggcgtgttg ctcgccagcc agaggagctc 840
   gcttattgta ccattgacgg caggggcaga gagggcaagg ggattccgtg gaagaagttt 900
   gactccaagg tggcggctgt ggccatgtat ctgaagaaca aagtcaaggt gcggccgggc 960
   gaccacctgg tcctcatgta cacccactcc gaggagtttg tctttgccgt ccacgcggga 1020
   atcaaccttg gcgcagtcat tattcccatg gcgccgcttg accagaaccg gctcaacgaa 1080
   gatgtccctg ctttcctgca cctgatcgct gactacaagg ttaaggcggt cctggtcaac 1140
   caggaagtgg accatttgct gaagctcaag atcgtgtcga gccacatcaa acagtccgca 1200
   cagatcctga agatctcgat gccgaatacc tacaacactt cgaagccacc taagcagaac 1260
   aacggtcttc gcgagcttgg gctgacgata gatcccgcct ggatcaggcc tggatacccc 1320
   gtcctcatct ggacatactg gacgccggac caacggagaa tcgccgtcca gctggggcat 1380
   gataccatcc tgggcatgtg caaagtgcag aaggagactt gtcagatgac gagcttccag 1440
   cccgttctcg gttgcgtaag aagcacaacg ggacttggtt tcgtgcacac gtgcctgatg 1500
   ggcatctacg ttggcaccgc cacctacctg ctgtctcctg tcgagttcgc ccaaaatccc 1560
   atctctctct ttgttacgtt gtcgaggtac aagatcaagg acacctatgc aacgccgcag 1620
   atgcttgacc atgccatgtc gtcgatgcag gccaagggct ttacaatgca cgaactgaag 1680
   aatatgatga ttactgcaga gggccggccg cgggtagatg tattccagaa ggtacggatg 1740
   cattttgcga gcgccgggct ggataggacg gccatcaaca cggtctactc gcatgtgctc 1800
   aacccgatga ttgcttcgag gtcttacatg tgcatcgagc ctattgagct ctggctcgac 1860
   accaaggctc ttcgacgcgg cctcgtcgtc ccggtcgatc acgattcaaa cccgcaagct 1920
   cttctcctgc aggattccgg catggtgccg gtgtctaccc agattgccat tgtcaacccc 1980
   gagagccgcg cgcattgcta cgatggagaa tatggcgaga tctgggtcga ctccgaggcg 2040
   tgcgtaaagg ccttttacgg ctccaaggaa gcgtttgacg tggagcgctt cgacggccgg 2100
   acggtcgacg gcgaccccaa cgtgcgatac gtgcgaactg gtgacttggg ctttttgtat 2160
   aatgtcaacc ggcctatcgg gcccaacggc gccctggtgg agatgcaact tgtttgtgct 2220
   cggtagcatc ggcgagactt ttgaaattaa cggtttgagt cacttcccca tggatattga 2280
   gctgtcggtg gaacgctgcc accgcaacat tgtacccaac ggctggtaag tacaggccaa 2340
   actcttctgt gagatgctac ttgactaata gttggtgatg tgcagtgctg tattccaagc 2400
   tggtggcttg gtcgtggtcc tggtagaggt gataacaaga cacagcccgg gctcttgtct 2460
   gttactgggc ctcatgggcc ttccgctcac tgcccgcttt ccagtcggga aacctgtcgt 2520
   gccagctgca ttaacatggt catagctgtt tccttgcgta ttgggcgctc tccgcttcct 2580
   cgctcactga ctcgctgcgc tcggtcgttc gggtaaagcc tggggtgcct aatgagcaaa 2640
   aggccagcaa aaggccagga accgtaaaaa ggccgcgttg ctggcgtttt tccataggct 2700
   ccgcccccct gacgagcatc acaaaaatcg acgctcaagt cagaggtggc gaaacccgac 2760
   aggactataa agataccagg cgtttccccc tggaagctcc ctcgtgcgct ctcctgttcc 2820
   gaccctgccg cttaccggat acctgtccgc ctttctccct tcgggaagcg tggcgctttc 2880
   tcatagctca cgctgtaggt atctcagttc ggtgtaggtc gttcgctcca agctgggctg 2940
   tgtgcacgaa ccccccgttc agcccgaccg ctgcgcctta tccggtaact atcgtcttga 3000
   gtccaacccg gtaagacacg acttatcgcc actggcagca gccactggta acaggattag 3060
   cagagcgagg tatgtaggcg gtgctacaga gttcttgaag tggtggccta actacggcta 3120
   cactagaaga acagtatttg gtatctgcgc tctgctgaag ccagttacct tcggaaaaag 3180
   agttggtagc tcttgatccg gcaaacaaac caccgctggt agcggtggtt tttttgtttg 3240
   caagcagcag attacgcgca gaaaaaaagg atctcaagaa gatcctttga tcttttctac 3300
   ggggtctgac gctcagtgga acgaaaactc acgttaaggg attttggtca tgagattatc 3360
   aaaaaggatc ttcacctaga tccttttaaa ttaaaaatga agttttaaat caatctaaag 3420
   tatatatgag taaacttggt ctgacagtta ccaatgctta atcagtgagg cacctatctc 3480
   agcgatctgt ctatttcgtt catccatagt tgcctgactc cccgtcgtgt agataactac 3540
   gatacgggag ggcttaccat ctggccccag tgctgcaatg ataccgcgag aaccacgctc 3600
   accggctcca gatttatcag caataaacca gccagccgga agggccgagc gcagaagtgg 3660
   tcctgcaact ttatccgcct ccatccagtc tattaattgt tgccgggaag ctagagtaag 3720
   tagttcgcca gttaatagtt tgcgcaacgt tgttgccatt gctacaggca tcgtggtgtc 3780
   acgctcgtcg tttggtatgg cttcattcag ctccggttcc caacgatcaa ggcgagttac 3840
   atgatccccc atgttgtgca aaaaagcggt tagctccttc ggtcctccga tcgttgtcag 3900
   aagtaagttg gccgcagtgt tatcactcat ggttatggca gcactgcata attctcttac 3960
   tgtcatgcca tccgtaagat gcttttctgt gactggtgag tactcaacca agtcattctg 4020
   agaatagtgt atgcggcgac cgagttgctc ttgcccggcg tcaatacggg ataataccgc 4080
   gccacatagc agaactttaa aagtgctcat cattggaaaa cgttcttcgg ggcgaaaact 4140
   ctcaaggatc ttaccgctgt tgagatccag ttcgatgtaa cccactcgtg cacccaactg 4200
   atcttcagca tcttttactt tcaccagcgt ttctgggtga gcaaaaacag gaaggcaaaa 4260
   tgccgcaaaa aagggaataa gggcgacacg gaaatgttga atactcatac tcttcctttt 4320
   tcaatattat tgaagcattt atcagggtta ttgtctcatg agcggataca tatttgaatg 4380
   tatttagaaa aataaacaaa taggggttcc gcgcacattt ccccgaaaag tgccac 4436

## Description

The present invention relates to a process for the production of a technical enzyme composition with low viscosity produced by a genetically modified filamentous fungus cell, a genetically modified filamentous fungus cell suitable for production of the technical enzyme composition, the use of such a genetically modified filamentous fungus cell for the production of the technical enzyme composition with low viscosity and a technical enzyme composition with low viscosity produced by such a process.

Enzymes are important components of many commercial products and respective production processes. Modern laundry compositions contain a wide variety of different enzymes such as cellulases, many feed products for livestock contain enzymes and enzymes are also used for the production of many commercial products such as the production of bioethanol, of plastic alternatives / biodegradable plastics or even food products. Enzymes used in such processes are often called "industrial enzymes" or "technical enzymes".

To attain economic feasibility of the desired end product, a high yield and low production cost of the used technical enzyme(s) is a necessity. This applies in particular when the desired commercial end product is a bulk product which has to compete with low price alternatives originating from cheap mineral-oil derived chemical synthesis processes.

Filamentous fungi are well known as effective producers of a wide variety of technically feasible enzymes. In addition, filamentous fungi are able to grow on a diverse range of substrates.

However, the implementation of filamentous fungi for the production of technical enzymes is still not very popular as the high viscosity of the fermentation broth of such fungi often affords time and cost consuming measures leading to too high production costs of the technical enzyme composition. In order to obtain a high yield of enzymes, a strong growth of the fungus is desired, however, strong growth results in a high content of fungus biomass within the fermentation broth. Fungi, which are known to consist of i.a. hyphae are known within the art as rendering any fermentation substrate into a high-viscous composition. This effect is significantly more distinct when a filamentous fungus is used which exhibits a sponge-like, slimy appearance.

High viscosity causes many problems, as the fungus needs constant oxygen supply by aeration during growth. In addition, cooling of the fermenter, especially in industrial-scale production is required. Both can only be guaranteed by constant stirring - on the one hand to distribute the air bubbles homogenously within the broth, and on the other hand to facilitate constant heat-exchange with the cooling devices. The higher the viscosity of the broth the more energy needs to be spent to realize effective stirring within the reactor. Further, more air has to be pressed into the reactor causing also higher energy consumption within the compressor and sterile-filter unit. Thus, both CAPEX and OPEX increase with increasing viscosity of the fermentation broth. An alternative measure - less cell mass production - is also not attractive for commercial production as this would always be accompanied by a lower yield of technical enzyme production.

The inventors of the present invention have therefore set themselves the task to develop a process for the production of a technical enzyme composition with low viscosity produced by a filamentous fungus while maintaining a high yield of enzymes.

The task has been solved by a process for production of a technical enzyme composition, comprising the following steps:
(a) providing a fermentation medium with a glucose content of from 5 to 450 g/L;
(b) addition of at least one filamentous fungus cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

It is of particular advantage of the inventive process that a high yield of target enzymes is achieved with any kind of medium which contains a significant amount of glucose. The majority of the proteins secreted by filamentous fungi are enzymes that degrade naturally occurring polymers such as cellulose and hemicellulose and the availability of glucose would usually prevent the filamentous fungus from producing such enzymes as they are not needed for metabolization of glucose. Further, no addition of expensive inducing substances such as gluco-oligosaccharides or sophorose is necessary. Therefore, a wide variety of different fermentation substrates which are readily and cheaply available may be used.

Within the present invention the term "technical enzyme composition" is to be understood to consist of or to contain a partly or completely fermented medium and may even contain components of the original medium but also any compound generated during the fermentation process such as enzymes. A "technical enzyme composition" may also contain part of or all of the microbial biomass of the fermentation microorganism i.e. the filamentous fungus.

Within the present invention the technical enzyme composition preferably contains at least one enzyme belonging to the class of hydrolases and/or at least one enzyme belonging to the class of oxidoreductases. Within a particularly preferred embodiment of the present invention, the technical enzyme composition contains at least one enzyme belonging to the class of hydrolases and/or at least one enzyme belonging to the class of oxidoreductases which has been produced by the at least one filamentous fungus cell. Within another also particularly preferred embodiment, the technical enzyme composition contains at least one enzyme belonging to the class of cellulases and/or at least one enzyme belonging to the class of hemicellulases which has been produced by the at least one filamentous fungus cell.

Within the present invention, the term "enzyme belonging to the class of hydrolases" is to be understood as comprising any enzyme, capable of the hydrolysis of a chemical bond. Enzymes belonging to the class of hydrolases are classified as EC 3 in the EC number classification of enzymes. According to the present invention, the term "hydrolases" comprises cellulases, hemicellulases and may also encompass pectinases, oxidases, chitinases, chitosanases, transglutaminases, pentosanases, niringinases, limoninases, lactonases, nucleases, ureases, lipoxygenases, esterases, alpha-glucanases, phosphatases, isomerases, proteases and accessory proteins.

Within the present invention, the "enzyme belonging to the class of hydrolases" may be a native enzyme of the filamentous fungus or a heterologous enzyme originating from a different species of microorganism, in particular from a different species of filamentous fungus but may also originate from a non-filamentous fungus or a bacterium.

As used within the present invention, the term "cellulase" refers to any enzyme capable of hydrolyzing cellulose polymers to shorter oligomers and/or glucose. Cellulases preferred within the technical enzyme composition include cellobiohydrolases (CBH) (EC 3.2.1.-), endo-1,4-β-glucanases (EG) (EC 3.2.1.4).), beta-glucosidase (EC 3.2.1.4), cellobiose hydrolase (EC 3.2.1.21), glycoside hydrolase 61 (GH61 and CBM33).

As used within the present invention, the term "hemicellulase" refers to any enzyme capable of degrading or supporting the degradation of hemicellulose. Hemicellulases preferred within the technical enzyme composition include β-glucanases (EC 3.2.1.-), endo-xylanases (EC 3.2.1.8), β-xylosidases (EC 3.2.1.37), acetylxylan esterase (EC 3.1.1.72), acetylgalactan esterase (3.1.1.6), acetyl mannan esterase, feruloyl esterase (EC 3.1.1.73), glucuronoyl esterase (EC 3.1.1.-), α-L-arabinofuranosidase (EC 3.2.1.55), α-arabinopyranosidase (3.2.1.-), α-galactosidase (EC 3.2.1.22), β-galactosidase (EC 3.2.1.23), α-glucuronidases (EC 3.2.1.139), β-mannase (EC 3.2.1.78), β-mannosidases (EC 3.2.1.25), mannan 1,4-mannobiosidase (EC 3.2.1.100), arabinogalactan endo-beta-1,4-galactanase (EC 3.2.1.89), endo-beta-1,3-galactanase (EC 3.2.1.90), galactan endo-beta-1,3-galactanase (EC 3.2.1.181, glucuronoarabinoxylan endo-1,4-beta-xylanase (EC 3.2.1.136), alpha-L-fucosidase (EC 3.2.1.51), coniferin beta-glucosidase (EC 3.2.1.126), xyloglucan hydrolases (EC 3.2.1.150, 151, 155), xylan α-1,2-glucuronosidase (EC 3.2.1.131), endo-xylogalacturonan hydrolase (EC 3.2.1.-; GH28), α-amylase (EC 3.2.1.1), glucan 1,4-α-glucosidase (EC 3.2.1.3), galactan 1,3-galactosidase (GH43), -1,4,-endogalactanase (EC 3.5.1.89; GH53), α-rhamnosidase (EC 3.2.1.40) and β-rhamnosidase (EC 3.2.1.43).

As used within the present invention, the term "pectinase" refers to any enzyme capable of degrading or supporting the degradation of pectin. Pectinases preferred within the technical enzyme composition include polygalacturonases (EC 3.2.1.15, 67, 82; GH28 pectin methyl esterase (EC 3.1.1.11), pectin acetyl esterase (EC 3.1.1.-), rhamnogalacturonase (EC 3.2.1.-; GH28), rhamnogalacturonan acetylesterase (EC 3.1.1.86), rhamnogalacturonan galacturonohydrolase (EC 3.2.1.-), xylogalacturonan hydrolase (EC 3.2.1.-), pectin methylesterase (EC 3.1.1.11), beta-arabinofuranosidase (EC 3.2.1.55), beta-1,4-galactanase (EC 3.2.1.89), beta-1,3-galactanase (EC 3.2.1.90), beta-galactosidase (EC 3.2.1.23), alpha-galactosidase (EC 3.2.1.22), feruloyl acetyl esterase (EC 3.1.1.-), alpha-fucosidase (EC 3.2.1.51), (beta-fucosidase) (EC 3.2.1.38), beta-apiosidase (EC 3.2.1.-), alpha-rhamnosidase (EC 3.2.1.40), beta-rhamnosidase (EC 3.2.1.43), alpha-arabinopyranosidase (EC 3.2.1.-), beta-glucuronidase (EC 3.2.1.31), alpha-glucuronidase (EC 3.2.1.139), beta-xylosidase (EC 3.2.1.37) and alpha-xylosidase (EC 3.2.1.x).

As used within the present invention the term "accessory protein" refers to any enzyme capable of supporting cellulolytic enzyme activity. The term is well known to a person skilled in the art. Preferred accessory proteins within the technical enzyme composition include Expansin, Swollenin, Loosenin and CIP Proteins (EC 3.1.1.-; CE15).

As used within the present invention, the term "oxidoreductase" refers to any enzyme capable of catalyzing an oxidation and/or a reduction reaction. Enzymes belonging to the class of oxidoreductases are classified as EC 1 in the EC number classification of enzymes. Oxidoreductase enzymes preferred within the technical enzyme composition include lytic polysaccharide monooxygenase (LPMO) (AA9-11; previously GH61 and CBM33, resp.) (EC 1.14.99.53-56, 1.14.99.B10), lignin peroxidase (EC 1.11.1.14), manganese peroxidase (EC 1.11.1.13), aryl-alcohol oxidase (EC 1.1.3.7), glyoxal oxidase (EC 1.1.3.), carbohydrate oxidases (EC 1.1.3.4, 9, 10), cellobiose dehydrogenase (EC 1.1.99.18), catalase (hydrogenperoxide oxidoreductase) (EC 1.11.1.6 or EC 1 .11.1.21), dye-decolorizing peroxidase (EC 1.11.1.19), laccase (EC 1.10.3.2), peroxidase (EC 1.11.1.x) and versatile peroxidase (EC 1.11.1.16).

As used within the present invention, the term "esterases" refers to any enzyme capable of cleaving an ester bond. Esterases preferred within the technical enzyme composition include acetyl esterases, glucuronoyl esterases, feruoyl esterases, lipases, cutinases and phospholipases.

As used within the present invention, the term "alpha-glucanases" refers to any enzyme capable of degrading alpha-linked oligo- and polysaccharides. Alpha-glucanases preferred within the technical enzyme composition include alpha-amylases, glucoamylases, pullulanases, dextranases, trehalases, lactases, invertases and maltases.

As used within the present invention, the term "phosphatase" refers to any enzyme capable of cleaving phosphoester bonds. Phosphatases preferred within the technical enzyme composition include phytases.

As used within the present invention, the term "isomerases" refers to any enzyme capable of transferring a chemical compound into an isomeric structure. Isomerases preferred within the technical enzyme composition include xylose isomerases, glucose isomerases and arabinose isomerases.

As used within the present invention, the term "proteases" refers to any enzyme capable of cleaving a peptide bond. Proteases preferred within the technical enzyme composition include serine proteases, threonine proteases, aspartic proteases, cysteine proteases, glutamic proteases and metalloproteases.

The enzymes referenced within the present invention are classified according nomenclatures that are either based on the International Union of Biochemistry and Molecular Biology's Enzyme Nomenclature and Classification (http://www.chem.qmul.ac.uk/iubmb/enzyme/) or on Carbohydrate-Active EnZYmes (http://www.cazy.org/) database.

According to the present invention the term "fermentation medium" is to be understood as referring to any fermentation medium known to a person skilled in the art as suitable for the inventive process. Within the process of the present invention, the fermentation medium contains from 5 to 450 g/L glucose, wherein glucose contents from 5 to 420 g/L, from 8 to 400 g/L and from 10 to 280 g/L are preferred. Further preferred ranges of glucose are from 10 to 450 g/L, from 40 to 400 g/L and from 50 to 350 g/L. Also preferred ranges of glucose are from 5 to 50 g/L, from 6 to 40 g/L or from 7 to 35 g/L and from 50 to 450 g/L, from 80 to 400 g/L and from 100 to 380 g/L. The glucose contained in the fermentation medium may originate from any source known to a person skilled in the art as suitable for the inventive process. Within a preferred embodiment, the glucose originates from corn, sugar cane or sugar beets, preferred sources are corn syrup, sugar cane or sugar beet molasses and mixtures thereof.

Within a preferred embodiment of the present invention, the fermentation medium further contains xylose and wherein the glucose to xylose ratio is selected from the range of from 1 to 3.5., such as a ratio selected from the range of from 1 to 3, from 1 to 2.8, of from 1 to 2.5 or of from 1 to 2.2. Further preferred ratios are 2.1, 2.0, 1.9 and 1.8.

Within an alternative preferred embodiment of the present invention, the fermentation medium further contains lactose and wherein the glucose to lactose ratio is selected from the range of from 1 to 10, such as a ratio selected from the range of from 1 to 9, from 1 to 8.5, of from 1 to 8 or of from 1 to 7. Further preferred ratios are 3, 4, 5 and 6.

Within a preferred embodiment of the present invention no gluco-oligosaccharides have been added to the fermentation medium and it is particularly preferred that the fermentation medium is free from gluco-oligosaccharides.

Within a preferred embodiment of the present invention no sophorose has been added to the fermentation medium and it is particularly preferred that the fermentation medium is free from sophorose.

Within another preferred embodiment of the present invention the fermentation medium contains less than 100 g/L cellulose and/or hemicellulose, preferably less than 80 g/L, more preferred less than 70 g/L, even more preferred less than 60 g/L, particularly preferred less than 50 g/L, and most preferred less than 40 g/L cellulose and/or hemicellulose. Within another preferred embodiment the fermentation medium of the present invention is free from hemicellulose. Within a further preferred embodiment of the present invention the cellulose content of the fermentation medium is selected from the range of from 0.01 g/L to 50 g/L, preferably from 0.1 to 40 g/L, further preferred of from 1 to 30 g/L and most preferred of from 1 to 20 g/L.

Within another preferred embodiment the fermentation medium has a nitrogen content of from 0.05 to 2.0 g/L. Preferred contents of nitrogen are selected from the range of from 0.1 to 1.5 g/L, from 0.3 to 1.2 g/l or from 0.5 to 1.0 g/L. The nitrogen can be added in any form known to a person skilled in the art as suitable for the inventive purpose and may be added in form of ammonium sulfate, ammonia, urea, extracts from soy beans or combinations thereof. The amount of nitrogen can be added by feeding or by adding the total amount to the fermentation medium at any time before or during step (a) and/or (b) of the inventive process. It is thereby preferred that the nitrogen is added as a 25% (wt.-/wt.) solution of ammonia or a 40 % (wt./wt.) solution of urea.

Within another preferred embodiment of the present invention, the fermentation medium contains from 0.5 to 80 wt.-% molasses, corn syrup or mixtures thereof, preferably from 5 to 75 wt.-%, from 15 to 70 wt.-%, from 25 to 65 wt.-%, from 35 to 60 wt.-% from 38 to 55 wt.-% or from 40 to 52 wt.-%.

Within a preferred embodiment of the inventive process the pH of the fermentation medium has been adjusted to a pH selected from the range of from pH 2.0 to pH 6.0, wherein ranges of from pH 3.0 to 5.5 and from pH 3.5 to 5.5 as well as from pH 3.5 to 5.0 are particularly preferred. The adjusting of the pH can be carried out by any means and method known to a person skilled in the art as suitable for the inventive purpose. Within the process of the present invention the pH is preferably adjusted by addition of an acid such as sulfuric acid or acetic acid, NaOH, H₃PO₄ or ammonia.

Within a preferred embodiment of the inventive process the fermentation medium has a potassium hydrogen phosphate content of from 0.5 to 10.0 g/L, a magnesium sulfate heptahydrate content of from 0.05 to 1 g/L, a calcium chloride dihydrate content of from 0.1 to 1 g/L, an ammonium sulfate content of from 1.5 to 4.5 g/L, an iron (II) sulfate heptahydrate content of from 0.005 to 0.1 g/L, a manganese sulfate content of from 0.00001 to 0.001 g/L, a zinc sulfate heptahydrate content of from 0.001 to 0.01 g/L and/or a copper sulfate pentahydrate content of from 0.0001 to 0.001 g/L. Further preferred ranges are potassium hydrogen phosphate content of from 1 to 8.0 g/L, a magnesium sulfate heptahydrate content of from 0.1 to 0.8 g/L, a calcium chloride dihydrate content of from 0.3 to 0.8 g/L, an ammonium sulfate content of from 1.7 to 4.0 g/L, an iron (II) sulfate heptahydrate content of from 0.01 to 0.9 g/L, a manganese sulfate content of from 0.0001 to 0.0008 g/L, a zinc sulfate heptahydrate content of from 0.002 to 0.008 g/L and/or a copper sulfate pentahydrate content of from 0.0002 to 0.008 g/L.

The "providing" of the fermentation medium according to step (a) of the inventive process can be carried out by any method and within any means known to a person skilled in the art as suitable for the inventive process. Within a preferred embodiment the fermentation medium is provided within a batch or fed batch reactor which is preferred equipped with a stirring device and a cooling device.

According to step (b) of the inventive process, at least one filamentous fungus cell wherein SEQ ID NO: 1 has been disrupted is added to the fermentation medium. The addition can be carried out by any means and measure known to a person skilled in the art as suitable for the inventive process. Within a preferred embodiment, the at least one filamentous fungus cell is added in a quantity of from 10² to 10¹⁰ cells, preferably in a quantity of from 10³ to 10⁸ cells and most preferred in a quantity of from 10⁴ to 10⁷ cells per g of fermentation medium. The at least one filamentous fungus cell can thereby be added in dried form, as conidia or in form of a preculture, containing rest of preculturing medium. It is also possible to add the at least one filamentous fungus cell in form of a fully cultured medium (also referred to as main culture).

Within the present invention the term "filamentous fungus cell" is to be understood as any cell from any filamentous fungus existing in nature and/or known to a person skilled in the art. The term also comprises any filamentous fungus cell either of natural origin or modified. The term "modified" refers to genetically and non-genetically modified fungi. i.e. fungi which have been modified by genetic methods (e.g. transformation) and non-genetic methods e.g. chemical mutagenesis or irradiation, both of which are known to those skilled in the art. Within a preferred embodiment the at least one filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes,* wherein *Trichoderma* and *Aspergillus* are particularly preferred, most preferred is *Trichoderma reesei* (teleomorph: *Hypocrea jecornia*).

Within another preferred embodiment of the present invention, the at least one filamentous fungus cell is a genetically modified filamentous fungus cell with the ability to express at least one heterologous hydrolyase or oxidoreductase enzyme, such as but not limited to an enzyme belonging to the class of cellulases, belonging to the class of beta-glucosidases or belonging to the class of xylanases or belonging to the class of lytic polysaccharide monooxygenases. Within such a preferred embodiment, the at least one heterologous hydrolase or oxidoreductase enzyme preferably originates from another filamentous fungus such as - but not limited to - *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.* Within a particularly preferred embodiment the at least one filamentous fungus cell is a *Trichoderma reesei* cell and the at least one heterologous hydrolase or oxidoreductase enzyme originates from *Acremonium, Ajellomyces, Alternaria, Armillaria, Arthroderma, Aspergillus, Bionectria, Bipolaris, Ceriporiopsis, Chaetomium, Cladophialophora, Clohesyomyces, Colletotrichum, Coniochaeta, Coniosporium, Diaporthe, Dothistroma, Emericella, Epicoccum, Exophiala, Fomes, Fonsecaea, Fusarium, Gibberella, Grosmannia, Hebeloma, Hortaea, Humicola, Hypocrea, Hypoxylon, Irpex, Isaria, Kuraishia, Leucoagaricus, Madurella, Magnaporthe, Marssonina, Metarhizium, Moniliophthora, Myceliophthora, Mycosphaerella, Neurospora, Oidiodendron, Ophiostoma, Paecilomyces, Paraphaeosphaeria, Penicillium, Phanerochaete, Phialophora, Pleurotus, Pochonia, Pseudocercospora, Pseudogymnoascus, Pyrenophora, Rasamsonia, Rhinocladiella, Rhizopus, Rhizosphaera, Rhynchosporium, Setosphaeria, Sphaerulina, Sporothrix, Stachybotrys, Stemphylium, Talaromyces, Termitomyces, Tilletiaria, Torrubiella, Trametes, Trichoderma, Trichophyton, Uncinocarpus* and/or *Valsa* species.

According to the present invention, the at least one filamentous fungus cell as is a filamentous fungus cell wherein SEQ ID NO: 1 has been disrupted. The "disruption" can thereby be carried out by any means and measure known to the person skilled in the art as suitable for the purpose of disruption. The term "disruption" comprises all techniques that either lead to the gene no longer being transcribed or to the protein encoded by the gene no longer being produced or only being produced in an inactive form.

Exemplary methods which can be used within the present invention are:
- the partial or complete removal from the genome of the gene, the region coding for the protein and/or the promoter or other regions necessary for the expression of the gene (= "deletion")
- the alteration of the DNA sequence of the coding region so that a shortened protein (= generation of a stop codon) and/or a protein with an altered amino acid sequence is produced which can no longer perform the function of the unchanged protein (= "mutation")
- the modification of the DNA sequence of the promoter or other regions necessary for the expression of the gene, so that the gene is no longer transcribed (= no RNA and therefore no protein is produced)
- the expression of RNA with a sequence complementary to that of the target gene. This leads to hybridization (= pairing of complementary sequences) of the two RNAs and to a degradation of this double-stranded RNA. As a result, no RNA of the target gene is available for protein synthesis (= RNA interference).

Within the present invention SEQ ID NO:1 is defined within the sequence protocol.

Mixing according to step (c) of the inventive process of the present invention is carried out for a time period from 1 minute to 10 days, preferably from 10 hours to 7 days, further preferred from 24 hours to 5 days, preferably under constant stirring with a power input from 150 to 20000 W/ m³ and more preferably from 500 to 15000 W/m³ and under oxygen controlled conditions. The average dissolved oxygen level is preferably selected from 0.01% to 80%, preferred from 0.1% to 50%, particularly preferred from 5% to 30% and most preferred from 12% to 28%. Within a particularly preferred embodiment, the dissolved oxygen level is controlled by a stirrer or compressed air flow or internal reactor pressure or a combination of two or three of these measures. Furthermore, mixing according to step (c) of the inventive process is carried out at a temperature of from 20 to 35 °C, preferably at a temperature of from 21 to 34 °C wherein a temperature selected from the range of from 22 to 33 °C is also preferred.

"Mixing" according to step (c) of the process of the present invention is preferably conducted in a batch mode (discontinuous), in a fed-batch mode or in a continuous mode. Most preferably, the inventive process is conducted in a fed-batch mode.

"Obtaining" according to step (d) of the inventive process is preferably carried out by harvesting the technical enzyme composition at the end of the time period applied for mixing during step (c) as it is without further treatment.

Within another preferred embodiment of the present invention, the inventive process further contains the step (e): subjecting the technical enzyme composition according to step d) to a purification method.The purification according to step (e) can be carried out by any measure known to a person skilled in the art as suitable for the inventive purpose. Suitable purification methods are selected from the group consisting of filtration (ultrafiltration, microfiltration, nanofiltration, depth filtration, sterile filtration, filter press), centrifugation, decantation, flotation, chromatographic separation, adsorption, electrodialysis, extraction, precipitation, crystallisation, spray drying, granulation, coating, extrusion or combinations thereof. Preferred are filter-based solid-liquid separations. It is further particularly preferred to use a filter press. The residues after the filtration should have a minimal solid content of 20 % (wt./wt.), preferably 25 % (wt./wt.), particularly preferred 30 % (wt./wt.) and most preferred 40 % (wt./wt.) solid content. In case the process according to the present invention involves solid-liquid separation as purification, the technical enzyme composition obtained according to step (d) of the inventive process is considered to be the liquid fraction.

Within a preferred embodiment of the inventive process, the process further comprises step
(ai) sterilization of the fermentation medium according to step (a).

Sterilization can thereby be carried out by any means or measure known to a person skilled in the art as suitable for the inventive purpose. Within a preferred embodiment, sterilization is carried out by filtration, such as but not limited to membrane filtration processes or by ultra high temperature heating. A combination of two or more sterilization methods is also possible, however, it is particularly preferred to only apply ultra high temperature heating (also referred to as UHT). The UHT treatment is preferably carried out at a temperature of from 100 to 155 °C and for a duration of from 10 to 30 seconds, more preferred at a temperature of from 120 to 140 °C for a duration of from 10 to 20 seconds.

Within another aspect, the present invention relates to a filamentous fungus cell wherein SEQ ID NO:1 has been disrupted. Disruption of SEQ ID NO:1 can be carried out by any means and measure known to a person skilled in the art to be suitable for the inventive purpose. Possible and preferred methods and measures have been defined within the description. Within a preferred embodiment, SEQ ID NO:1 has been disrupted by deletion, mutation, modification of a promotor or any other regulatory sequence, generation of a stop codon or RNA interference. The term "filamentous fungus cell" has been defined within the description. All definitions given apply.

Within a preferred embodiment, the filamentous fungus cell is a genetically modified filamentous fungus cell with the ability to express at least one heterologous hydrolase enzyme. Such genetically modified filamentous fungus cell has been defined within the description. Within a particularly preferred embodiment of the present invention, the filamentous fungus cell is a genetically modified filamentous fungus cell wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence.

In another aspect the present invention relates to a technical enzyme composition produced according to the process as defined before.

In a further aspect the present invention relates to the use of a filamentous fungus cell as defined before for the production of a technical enzyme composition as defined before.

### Generally preferred embodiments

In the following, generally preferred embodiments of the present invention are listed which do not limit the scope of the invention and/or scope of the claims in any respect. The generally preferred embodiments illustrate particularly suitable embodiments for the production of technical enzyme composition by the filamentous fungus *Trichoderma reesei.*

### Generally preferred embodiment 1

Process for production of a technical enzyme composition, comprising the following steps:
(a) providing a fermentation medium with a glucose content of from 5 to 450 g/L;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 2

Process for production of a technical enzyme composition, comprising the following steps:
(a) providing a fermentation medium with a glucose content of from 5 to 450 g/L and wherein the fermentation medium further contains xylose and wherein the glucose to xylose ratio is selected from the range of from 1 to 3.5;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 3

Process for production of a technical enzyme composition, comprising the following steps:
(a) providing a fermentation medium with a glucose content of from 5 to 450 g/L and wherein the fermentation medium is free from cellulose, hemicellulose, gluco-oligosaccharides and/or sophorose;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 4

Process for production of a technical enzyme composition, comprising the following steps:
(a) providing a fermentation medium with a glucose content of from 5 to 450 g/L and a cellulose content of from 0.01 g/L to 1 g/L;
(b) addition of at least one *Trichoderma reesei* cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 5

Process for production of a technical enzyme composition, comprising the following steps:
(a) providing a fermentation medium with a glucose content of from 5 to 450 g/L;
(b) addition of at least one *Trichoderma reesei* cell wherein SEQ ID NO:1 has been disrupted by deletion, mutation, modification of a promotor or any other regulatory sequence, generation of a stop codon or RNA interference and wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 6

Process for production of a technical enzyme composition, comprising the following steps:
(a) providing a fermentation medium with a glucose content of from 5 to 450 g/L and wherein the fermentation medium is free from sophorose and/or gluco-oligosaccharides;
(b) addition of at least one *Trichoderma reesei* cell wherein SEQ ID NO:1 has been disrupted by deletion, mutation, modification of a promotor or any other regulatory sequence, generation of a stop codon or RNA interference and wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence;
(c) mixing of the fermentation medium and the at least one filamentous fungus cell for a time period of from 1 minute to 10 days at a temperature of from 20 to 35 °C;
(d) obtaining a technical enzyme composition.

### Generally preferred embodiment 7

*Trichoderma reesei* cell, wherein SEQ ID NO:1 has been disrupted by deletion, mutation, modification of a promotor or any other regulatory sequence, generation of a stop codon or RNA interference, comprising at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase encoding sequence, at least one heterologous protease enzyme encoding sequence, at least one heterologous isomerase enzyme encoding sequence and/or at least one heterologous lytic polysaccharide monooxygenase enzyme encoding sequence.

### Generally preferred embodiment 8

*Trichoderma reesei* cell, wherein SEQ ID NO:1 has been disrupted by deletion, mutation, modification of a promotor or any other regulatory sequence, generation of a stop codon or RNA interference, comprising at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidase enzyme encoding sequence, at least one heterologous protease enzyme encoding sequence, at least one heterologous isomerase enzyme encoding sequence and/or at least one heterologous lytic polysaccharide monooxygenase enzyme encoding sequence and wherein the at least one heterologous enzyme sequence originates from *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes .*

### Generally preferred embodiment 9

Technical enzyme composition produced according to a process as defined by any of generally preferred embodiment 1 to 8.

### Generally preferred embodiment 10

Use of a filamentous fungus cell as defined by any of generally preferred embodiments 8 or 9 for the production of a technical enzyme composition.

### Figures and examples

The present invention is described by the following figures and examples. It is thereby emphasized that the figures and examples do not limit the scope of the invention and claims but merely constitute further illustration of the invention, inventive purpose and benefits achieved by the inventive method.

### List of figures

- Figure 1:: Protein concentrations in the culture supernatants of pSEQ1M-HygR transformants MSEQ1-1 to -3 and reference strain M18.2b. Values are given in relation to the average protein concentration in the supernatants of the host strain M18.2b which is set to 1.
- Figure 2:: Biomass concentrations in the culture broths of pSEQ1M-HygR transformants MSEQ1-1 to -3 and reference strain M18.2b. Values are given in relation to the average biomass concentration in the culture broth of the host strain M18.2b which is set to 1.
- Figure 3:: Viscosity of culture broths of pSEQ1 M-HygR transformants MSEQ1 -1 to -3 and reference strain M18.2b. Values are given in relation to the viscosity of the culture broth of the host strain M18.2b which is set to 1.
- Figure 4:: SDS-PAGE gel of culture supernatants of pSEQ1M-HygR transformants MSEQ1-1 to -3 and reference strain M18.2b. Reference bands ("Marker" lane) correspond to 250, 150, 100, 75, 50, 37, 25 and 20 kD

### General

The examples describe a way to disrupt the *Trichoderma reesei SEQ1* gene by deleting two nucleotides resulting in a frame shift and a change of an amino acid coding codon to a stop codon. They also show the effect of the SEQ1 gene disruption on the protein production, biomass formation and culture broth viscosity of *T. reesei.*

### Example 1: Mutation of SEQ NO:1

### Construction of a SEQ1 mutation vector

Standard methods known to those skilled in the art and described e.g. by Sambrook and Russel (Molecular Cloning - A laboratory manual; Cold Spring Harbor Laboratory Press, New York) or by Jansohn et al. (Gentechnische Methoden, Elsevier, München) were used for DNA agarose gel electrophorese, purification of DNA, transformation of *Escherichia coli,* plasmid propagation and purification, amplification of pieces of DNA by polymerase chain reaction (PCR) and isolation of genomic DNA from *Trichoderma reesei.* Ligation-independent cloning (LIC) was done essentially as described by Aslanidis and de Jong (1990, Nucleic Acid Res. 18 (20), 6069).

A SEQ1 mutation vector was constructed by fusing the Hygromycin B resistance marker to the SEQ1 3' flanking region and cloning the fusion product in a plasmid containing a part of the SEQ1 coding region that introduces a mutation encompassing the deletion of the nucleotides G4060 and T4061 (positions according to SEQ ID NO: 1) into the S*EQ1* gene.

The Hygromycin B resistance marker cassette (SEQ ID NO:2) had been synthesized by Thermo Scientific. Primers hygrfw (5'- TGCAAGGCGATTAAGTTGGG -3') and hygrrv (5'-CGGCGAGGATCTTTCCTCGCTGCTTCTCTCAACAGACAAGAGCCCTATAACTTC -3') were used to amplify the approximately 2.6 kb long cassette (annealing temperature: 63.2 °C, elongation time: 1 min, 30 cycles) using phusion polymerase from Thermo Scientific.

Genomic DNA from *Trichoderma reesei* M18.2b (DSM 19984) was isolated and used as a template together with the primers SEQ1fl3fw (5'-TTGTCAACGCCATCTTGAGC -3') and SEQ1fl3fw (5'-ACCAACCAGTCCATCCTCTG -3') to amplify an approximately 2.2 kb 3' flanking fragment of SEQ1 (annealing temperature: 64.5 °C, elongation time: 1 min 15 sec, 30 cycles) using phusion polymerase from Thermo Scientific.

The PCR-amplified hygromycin B resistance marker cassette and SEQ1 3' flanking region were purified and fused using phusion polymerase from Thermo Scientific and the primers fus1 (5'- AAACCAGACAGACAGTATACGACTCACTATAGGGCG -3'), fus2 (5'-GTTAACAGACAAGAGCCCGAAGTTATTCGGGTAGTAGAGTTTGAAAGGGG -3') und fus3 (5'- AGAGAGGAGAGACAGTGTTAACAGACAAGAGCCCGAAG -3'). Approximately 100 ng of both templates, 20 µM of primers fus1 and fus3 and 2 µM of primer fus2 were used. The PCR consisted of 10 initial cycles of 10 sec at 98 °C, 30 sec at 65 °C and 1 min 20 sec at 72 °C followed by cooling to 10 °C. Then the primers were added, followed by a 30 sec hold at 98 °C and 30 cycles of 10 sec at 98 °C, 30 sec at 61.5 °C and initial 2 min 5 sec at 72 °C with the 72 °C incubation being extended by 5 sec per cycle. The PCR was concluded by a 10 sec hold at 72 °C and cooling to 10 °C.

The approx. 4.1 kb long fusion PCR product was purified and cloned into a *PshAl-*linearized pUC19-derived plasmid (SEQ ID NO: 3) that contained a LIC reception site instead of the multiple cloning site. The linearized vector was treated with T4 DNA polymerase in the presence of dTTP. The fusion PCR product was treated with T4 DNA polymerase in the presence of dATP. T4 DNA polymerase treated vector and fusion PCR amplicon were mixed and annealed as described in by Aslanidis and de Jong. The LIC assay was then transformed in chemically competent *Escherichia coli* XL1-Blue cells (Agilent), plated on LB-Agar plates containing 100 mg.l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by digestion with *Hpa*I*.* Plasmid clones were verified by Sanger sequencing and one plasmid with correct sequence was designated pSEQ1-3fl-HygR.

Plasmid pSEQ1flank5 (synthesized at Thermo Scientific; SEQ ID NO: 4), containing a modified part of the SEQ1 gene that introduces a mutation encompassing the deletion of the nucleotides G4060 and T4061 (positions according to SEQ ID NO: 1) into the SEQ1 gene was digested with S*rf*I (New England Biolabs).

The Hygromycin resistance marker - SEQ1 3' flanking region fragment (approx. 4.0 kb) was released from pSEQ1-3fl-HygR by restriction digestion with *Hp*aI. The *Srf*I-linearized vector pSEQ1flank5 was treated with T4 DNA polymerase in the presence of dTTP. The 4.0 kb *H*paI fragment from pSEQ1-3fl-HygR was treated with T4 DNA polymerase in the presence of dATP. T4 DNA polymerase treated vector and insert were mixed and annealed as described in by Aslanidis and de Jong. The assay was then transformed in chemically competent *Escherichia coli* XL1-Blue cells (Agilent), plated on LB-Agar plates containing 100 mg.l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by digestion with *Xmn*I. Plasmid clones were verified by Sanger sequencing and one plasmid with correct sequence was designated pSEQ1 M-HygR.

### Transformation of the SEQ1 mutation vector into Trichoderma reesei

Vector pSEQ1 M-HygR was digested with *Xmn*I (New England Biolabs) according to the manufacturer's instructions and the mutation cassette (6.0 kb) was purified by agarose gel electrophoresis and with the Wizard PCR purification kit from Promega. *Trichoderma reesei* M18.2b (DSM 19984) was transformed with the digested vector essentially as described in Penttilä et al. (1987) Gene 61: 155-164 or Gruber et al. (1990) Curr Genet 18: 71-76. The transformants were selected on potato dextrose agar plates containing 100 mg.l⁻¹ of Hygromycin B and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g·l⁻¹)-Triton X-100 (0.01 g.l⁻¹) solution, adjusted to OD₆₀₀ = 10 with sterile water, supplemented with glycerol to a final concentration of 50 g.l⁻¹ and stored at -80 °C.

Genomic DNA was isolated from the mycelium of the transformants and the host strain. The integration of the *SEQ1* mutation cassette at the intended locus was verified by PCR using phusion polymerase from Thermo Fisher Scientific according to the manufacturer's instructions, genomic DNA from the transformants as template and primers SEQ1MKOfw (5'- ATGTGCTAGGATTGTACGAG -3') and SEQ1MK01rv (5'- ATAATAGCTCATGGTCTCAC -3') (annealing temperature: 57.3 °C, elongation time: 1 min 20 sec, 30 cycles) or primers SEQ1 MKOfw (5'-ATGTGCTAGGATTGTACGAG -3') and SEQ1MKO2rv (5'-TTGACAAAGGCCACAATATC -3') (annealing temperature: 59.3 °C, elongation time: 1 min 15 sec, 30 cycles), respectively. A 2.6 kb band with primers SEQ1 MKOfw and SEQ1MKO1rv indicates the integration of the mutation cassette at the SEQ1 locus, while a 2.4 kb band with primers SEQ1 MKOfw and SEQ1 MKO2rv indicates that the *SEQ1* locus is still native (i.e. this band was not expected with genomic DNA from transformants that had integrated the pSEQ1M-HygR fragment at the intended locus). Genomic DNA from strain M18.2b was also tested as a control. In order to verify that the intended mutation had been inserted into the *SEQ1* ORF, the amplicon obtained with primers SEQ1 MKOfw and SEQ1MKO1rv was sequenced using primer M1Seq-01 (5'- ATCGCTACTTCTTTGTTCAG -3') and M1Seq-02 (5'-CAGCTTGGAATACAGCACTG -3').

Three transformants containing the mutation from pSEQ1M-HygR in the *SEQ1* ORF were named MSEQ1-1 to -3.

### Growth of the SEQ1 deletion strains in shake flasks

The strains MSEQ1-1 to -3 and M18.2b were grown in shake flasks in Medium 1. Medium 1 contains (g.l⁻¹):

| Name | Concentration [g/l] |
|---|---|
| Acetic acid | 0.34 |
| Calcium | 0.12 |
| Chloride, water soluble | 0.15 |
| Copper | 0.0001 |
| Fat (HCI soluble) | 0.001 |
| Furfural | 0.003 |
| Glucose | 6.5 |
| Glycerol | 0.009 |
| HMF | 0.006 |
| Iron | 0.004 |
| Magnesium | 0.048 |
| Manganese | 0.002 |
| Na-D/L-Lactat | 0.097 |
| Nitrogen, soluble | 0.85 |
| Phosphorus | 0.48 |
| Phthalate | 8.2 |
| Potassium | 3.2 |
| Sodium | 0.015 |
| Sulfur | 0.86 |
| Xylose | 3.6 |
| Zinc | 0.001 |

The medium was adjusted to pH 5.5 with HCI or NaOH and sterilized by autoclaving (20 min at 121 °C).

15 ml of the medium were distributed into 50 ml Erlenmeyer shake flasks under a sterile hood. Conidia stocks of strains MSEQ1-1 to -3 and M18.2b were thawed, 75 µl of the conidia suspensions were pipetted into the Erlenmeyer flasks with the medium under a sterile hood and the flasks were closed with rubber foam caps. Three flasks were inoculated per strain. The flasks were incubated at 30 °C with shaking (250 RPM) for 6 days. After 6 days, the cultures were poured into 15 ml tubes. Aliquots were removed, centrifuged (3220xg, 4 °C, 15 min) and the supernatants stored at 4 °C, while the remaining culture broth was used for determination of the biomass and viscosity (see below).

### Characterization of the culture supernatants and broths: Protein concentration, SDS-PAGE, Biomass, Viscosity

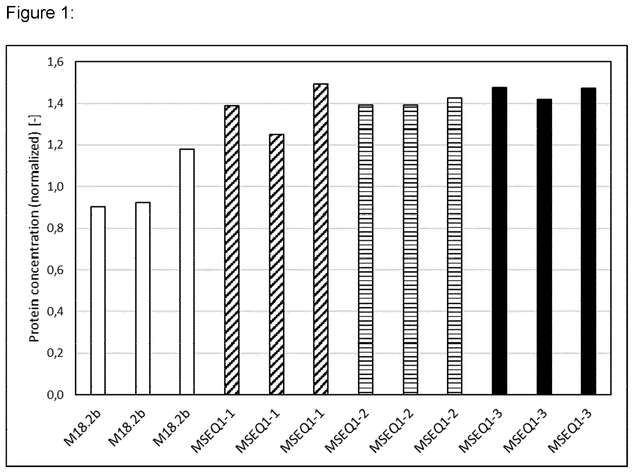

Protein concentrations in the centrifuged culture supernatants of strains MSEQ1-1 to -3 and M18.2b were measured using the Quick Start^{™} Bradford reagent (BioRad) and BSA standard solutions (BioRad) according to the supplier's instructions. The results of the measurements are shown in Figure 1. Values are given in relation to the average protein concentration in the supernatants of the host strain M18.2b which is set to 1. It is obvious from these data that strains MSEQ1-1 to -3 produce significantly more protein than the host strain M18.2b.

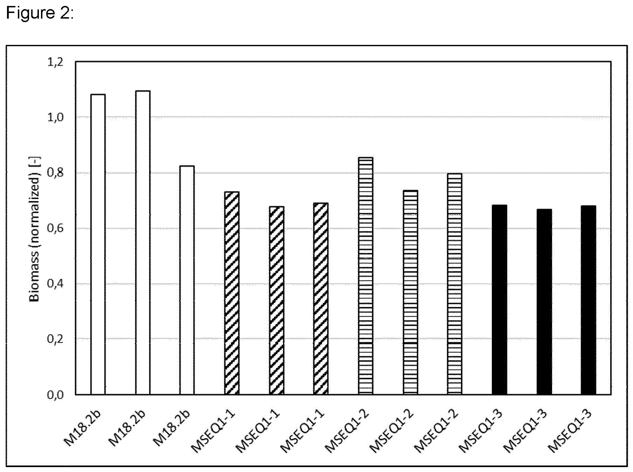

For biomass determination, Whatman^{™} filter discs (P1) were dried at 60 °C until their weight remained constant for 24 h, cooled to room temperature and weighed. Culture broths of strains MSEQ1-1 to -3 and M18.2b were filtered using those dried filter discs and the mycelia were washed with at least ten times the broth's volume of deionized water. Then the filter discs with the mycelia were dried at 60 °C until their weight remained constant for 24 h. The filter discs with the dried mycelia were weighed. The biomass concentration in the culture broth was then calculated by subtracting the mass of the dried filter disc from the mass of the dried filter disc with the mycelia and then dividing that value by the volume of the culture broth that had been filtered. The results of the measurements are shown in Figure 2. Values are given in relation to the average biomass concentration in the supernatant of the host strain M18.2b which is set to 1. It is obvious from these data that strains MSEQ1 -1 to -3 produce significantly less biomass than the host strain M18.2b

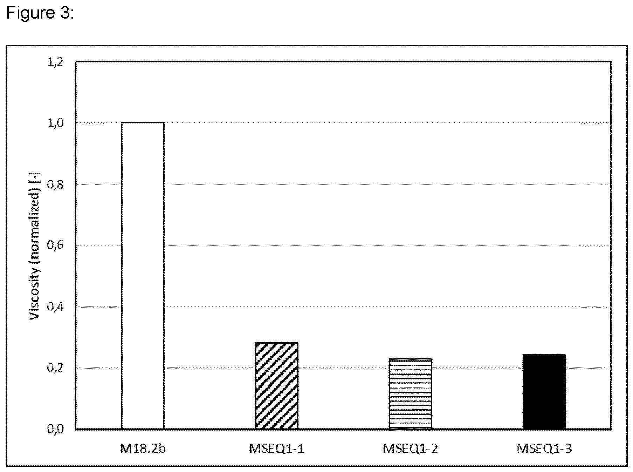

The viscosity of the culture broths of strains MSEQ1 -1 to -3 and M18.2b was measured using a Malvern Kinexus Lab+ KNX2110 rotational rheometer with the Vane tool (4Vnn:CUPnn) according to the manufacturer's instructions. The measurements were taken at a temperature of 20 °C and at a rotation velocity of 18.11 RPM ("rotations per minute"). The viscosity is depicted in Figure 3 and is presented in relation to the viscosity of the culture broth of strain M18.2b, which is set to 1. It is obvious from these data that the viscosity of the culture broths produced with MSEQ1-1 to -3 is significantly lower than that of the host strain M18.2b

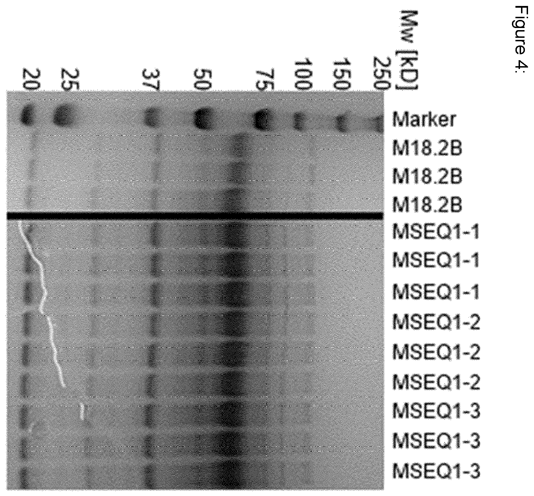

SDS-PAGE analysis of the centrifuged culture supernatants of strains MSEQ1-1 to -3 and M18.2b was done using methods known to those skilled in the art (e.g. described by Jansohn et al. (Gentechnische Methoden, Elsevier, München)) and the Criterion XT system (BioRad). Equal volumes of culture supernatants were loaded in each lane. Precision Plus Protein^{™} All Blue Standards (BioRad) was used as protein size reference. The gel image is shown in Figure 4. A person skilled in the art will recognize that the protein pattern of MSEQ1-1 to -3 is indistinguishable from that of the host strain M18.2b.

### Summary

Taken together these data demonstrate that the disruption of the SEQ1 gene results in a significantly more efficient protein production, with more protein and less biomass being formed. The analysis of the secreted proteins by SDS-PAGE shows that their composition doesn't change significantly, indicating a general increase in protein production. In addition, the viscosity of the culture broth is significantly reduced as well.

### Sequence listing

SEQ ID NO: 1

SEQ1 native gene

ATGGTTTCTGGCGACTACGCCTTCAACCCCGATCAACACGGCGCATATGCCGAA
CCGTACCAACAGCCGGACGACGGCCGGACTAGGACGCTGCTTGACAACCAAGC
CTTCTTTTCTGACTTCGCGGGCCAGCAGCACTACGAACAGAACCAGATGGGCG
ACTATGGTGGCCCTAGATACTCCGGCGATGCCTTCTCTCCGACAGCAGCCATG
GCTCCTCCGATGCTCACTGCCAACGACATGCCTCCACCCGAGATATTGGAGTAC
CAGGCTCCGCTCGAGCCAAGAGAGGTCCCCTTTGCCATTCAGGATCCCCACGA
CAACAACACGGCCATGTCTTCGTTCGACAACATGGCTGCGGTACTCCGTCACCG
TGCCCGCACCACTGCCAAAAGACCTGCATATTGGGTCCTGGACAGCAAGGGCA
AGGAGGTGGCATCCATTACATGGACAAGCTGGCGTCAAGAGCGGAGAAAGTT
GCACAAGTCATCCGAGACAAAAGTCCTCTTTACCGTGGCGATCGAGTTGCCTTG
ATCTATCGTGATAGCGAAATCATTGACTTCGCCATTGCCTTGCTGGGTTGCTTCA
TTGCTGGAGTTGTGGCTGTCCCGATCAATGACTTGCAAGACTACCAGCGCCTCA
ACTATATTCTCACCTCGACTCAGGCGCATCTGGCTCTTACTACCGAAAACAACCT
CAAGACCTTCCAGAGAGACATTACTGCGCAGAAGCTCACGTGGCCTAAAGGGG
TCGAGTGGTGGAAGACCAACGAGTTCGGCGGTTACCATCCGAAGAAGAAGGAA
GACGCACCTCCGTTAACTGTTCCCGACCTGGCCTATATTGAGTTTTCGCGAGCA
CCAACCGGCGACTTGAGGGGCGTTGTTCTCAGCCACAGGACAATCATGCACCA
GATGGCCTGCCTCAGTGCCATAATCTCTACCGTTCCCACCAACGGCCCCGGCG
ATACCTTCAACTCGACGTTGCGGGACAAGAACGGAAAGCTCATCGGCGGCGGA
GCCAGCAGCGAGATATTGCTCTCCTATCTGGATCCCCGACAGGGCGTGGGCAT
GATTCTCGGCGTTTTGCTGACCGTTTACGGCGGCCACACTACCGTCTGGTTCGA
TCACAAAGCCGTCGAGTCGCCTGGCTTATACGCGCATCTGATTACCAAGTACAG
AGCGACGATTATGATTGCGGATTACCCCGGGTTGAAGCGAGCTGCCTACAACTA
CCAGCAAGACCCCATGACGACACGAAACTTCAAAAAGGGGATGGAACCCAACTT
CCAAGCGGTGAAGCTGTGCTTGATTGATACCCTGACCATTGATAGCGAGTTCCA
TGAAGTTCTGGCCGATAGATGGCTGCGGCCCCTGCGAAATCCGCGAGCGCGC
GAGGTCGTGGCGCCGATGCTCTGCCTCCCCGAGCATGGCGGCATGATCATTAG
CGTTCGAGACTGGCTCGGCGGTGAAGAACGACTGGGAGTTCCGCTGAAACTGG
ACGAGTCTGACAGGGAGTCGGATGACGAGAAAGAAGAGGAAGAGAAGCCGGC

CCCGTCAAACGGATTTGGTAGCTTGCTTGGTGGTGGAGCAGCGACAACCAAGG
AGCAGGACGAGAAGATTGAGTTGGGCGAGGTTATCCTTGACCGAGAGGCTCTC
AAGACCAACGAGGTTGTCGTCTTGGCTCATGGCAACGAAGCTAGGAAGAAGAC
GTCGCTGGAGCCCACCATGGTCCGGGTCGGCGCCTTTGGATACCCTATCCCAG
ATGCCACGCTTGCTGTTGTGGACCCTGAGACTGGCCTCCTGGCAGCGCCGCAC
ACGATTGGCGAGATCTGGGTTGACTCTCCGTCTCTCTCTGGAGGCTTCTGGGC
GCAGCCAAAGAACACCGAGCTCATCTTCCACGCGCGTCCGTACAAGTTCGAGC
CTGGCGAGCCGACGCCAACTGCCGTGGAGCCGGAATTCCTGCGAACCGGCCT
GCTTGGCACAGTCATCGAGGGCAAGATCTATGTGCTAGGATTGTACGAGGATC
GGATACGACAAAAGGTCGAATGGGTTGAGCACGGCCACGCGGGTATCGCCGA
GTATCGCTACTTCTTTGTTCAGCACATTGTGGTGAGCATCGTCAAGAATGTCCCC
AAGATCCACGACTGCTCTGCCTTTGACGTCTTTGTCAATGACGAGCACTTGCCT
GTCGTGGTCCTCGAGTCTGCCGCAGCATCAACGGCGCCTCTCACTTCGGGCGG
CCCCCCTGTCCAGCCTGACACGGTTCTGTTAGACTCGCTGGCGGAGAAATGCA
TGGAGGTGCTCATGCAGGAGCACCATCTTCGGGTTTACTGCGTTATGATCACAG
CCCCGAACGCACTGCCGCGAGTGATCAAGAACGGAAGACGGGAAATAGGAAC
ATGCTCTGCCGGCGCGAGTTTGACCTTGGCAACCTCCCATGCGTGCATGTCAAA
TTTGGCGTCGAACATGCGGTTCTCAACCTCCCGATTGGCGTTGACCCCATTGGT
GGTATCTGGTCACCAATCGCCTCGGACTCGAGAATCAATATCCTGGCTCCCGCC
GATAAGCAGTATTCTGGAATCGACCGCAGAGAGGTTGTTATGGACGACCGGAC
GTCTACACCGCTCAACAATTTCAAGACCATCACCGATCTGATCCAGTGGCGTGT
TGCTCGCCAGCCAGAGGAGCTCGCTTATTGTACCATTGACGGCAGGGGCAGAG
AGGGCAAGGGGATTCCGTGGAAGAAGTTTGACTCCAAGGTGGCGGCTGTGGCC
ATGTATCTGAAGAACAAAGTCAAGGTGCGGCCGGGCGACCACCTGGTCCTCAT
GTACACCCACTCCGAGGAGTTTGTCTTTGCCGTCCACGCGGGAATCAACCTTGG
CGCAGTCATTATTCCCATGGCGCCGCTTGACCAGAACCGGCTCAACGAAGATGT
CCCTGCTTTCCTGCACCTGATCGCTGACTACAAGGTTAAGGCGGTCCTGGTCAA
CCAGGAAGTGGACCATTTGCTGAAGCTCAAGATCGTGTCGAGCCACATCAAACA
GTCCGCACAGATCCTGAAGATCTCGATGCCGAATACCTACAACACTTCGAAGCC
ACCTAAGCAGAACAACGGTCTTCGCGAGCTTGGGCTGACGATAGATCCCGCCT
GGATCAGGCCTGGATACCCCGTCCTCATCTGGACATACTGGACGCCGGACCAA
CGGAGAATCGCCGTCCAGCTGGGGCATGATACCATCCTGGGCATGTGCAAAGT
GCAGAAGGAGACTTGTCAGATGACGAGCTTCCAGCCCGTTCTCGGTTGCGTAA
GAAGCACAACGGGACTTGGTTTCGTGCACACGTGCCTGATGGGCATCTACGTT

GGCACCGCCACCTACCTGCTGTCTCCTGTCGAGTTCGCCCAAAATCCCATCTCT
CTCTTTGTTACGTTGTCGAGGTACAAGATCAAGGACACCTATGCAACGCCGCAG
ATGCTTGACCATGCCATGTCGTCGATGCAGGCCAAGGGCTTTACAATGCACGAA
CTGAAGAATATGATGATTACTGCAGAGGCCGGCCGCGGTAGATGTATTCCA
GAAGGTACGGATGCATTTTGCGAGCGCCGGGCTGGATAGGACGGCCATCAACA
CGGTCTACTCGCATGTGCTCAACCCGATGATTGCTTCGAGGTCTTACATGTGCA
TCGAGCCTATTGAGCTCTGGCTCGACACCAAGGCTCTTCGACGCGGCCTCGTC
GTCCCGGTCGATCACGATTCAAACCCGCAAGCTCTTCTCCTGCAGGATTCCGGC
ATGGTGCCGGTGTCTACCCAGATTGCCATTGTCAACCCCGAGAGCCGCGCGCA
TTGCTACGATGGAGAATATGGCGAGATCTGGGTCGACTCCGAGGCGTGCGTAA
AGGCCTTTTACGGCTCCAAGGAAGCGTTTGACGTGGAGCGCTTCGACGGCCGG
ACGGTCGACGGCGACCCCAACGTGCGATACGTGCGAACTGGTGACTTGGGCTT
TTTGTATAATGTCAACCGGCCTATCGGGCCCAACGGCGCCCTGGTGGAGATGC
AAGTCTTGTTTGTGCTCGGTAGCATCGGCGAGACTTTTGAAATTAACGGTTTGA
GTCACTTCCCCATGGATATTGAGCTGTCGGTGGAACGCTGCCACCGCAACATTG
TACCCAACGGCTGGTAAGTACAGGGCCAACTCTTCTGTGAGATGCTACTTGACT
AATAGTTGGTGATGTGCAGTGCTGTATTCCAAGCTGGTGGCTTGGTCGTGGTCC
TGGTAGAGGTGAGCCGCAAGTCTTACCTCGCCTCCATGGTGCCAGTCATTGTCA
ACGCCATCTTGAGCGAGCATCAGATCGTTGCCGATATTGTGGCCTTTGTCAACA
AGGGCGACTTCCCACGCTCTCGCCTGGGAGAGAAGCAGCGAGGAAAGATCCTC
GCCGGATGGGTTACGCGCAAGATGCGCACCATGGCCCAGTTCGCCATCAGAGA
TCTCGACGCCAGCATGCTCGAGCCGGGTGAGGGTCCGGATGCCAATAGGACCT
CTACGGGTAGCCTCCGTAGCCTGGGCGCCGCCGTCCCGCCAAACTTCAAGATG
GTTGGACAGGCGCCGCAGATACTGGAACAGGAGGAATTTACGCAGCAGATGGA
TCACATGGCCCATTCGGAGCCGGTCAGGCATGCCATGGCCGCTCCAGAGGAGC
AGCAGGCGCCGGCGGCCTATTACGCTGGCGGCCAGGAGGCTGCTTTCATGCA
GGGCTATAATCAGCAGCCGCCACCACCACCAAGCAGCCAGGGAGGATACC
AGTACGAGCAATTCGAGCCAGTGCAAGCACAGCAACAGTACCAGCCGCAGTCA
CAGCATCAGTTTGAACCATCTCAAGCCTTTGAGCCAGCGCAGCAATACGAGCCA
GCGCAACAGCACGAGCAGGAGCCAAGGCCGATGGACTCTCGAGGACAAGATG
CGCCGTCCATTGTTGAGCCAGAGACCTCAGCCTCCGTGCCTGATACGCCGCCG
CCGAGAAACAGGTTGAGTCAAGGGCCGCCCCAGATCCGGCTCCCGGGCGTTG
ACGGACGGGAAGGGCTCGACTTCTGGGGAGGCAACGACGAGACGGACTGGAC
GGCCGATGCCATGATGCACATGAATCTCACTGGCCCACGGTAA

SEQ ID NO: 2

Hygromycin B resistance marker

TGCAAGGCGATTAAGTTGGGTAACGCCAGGGTTTTCCCAGTCACGACGTTGTAA
AACGACGGCCAGTGAGCGCGACGTAATACGACTCACTATAGGGCGAATTGGCG
GAAGGCCGTCAAGGCCTAGGCGCGCCATGAGCTCGTTAACAAGACACAGCCCT
ATAACTTCGTATAATGTATGCTATACGAAGTTATATAACGGTGAGACTAGCGGCC
GGTCCCCTTATCCCAGCTGTTCCACGTTGGCCTGCCCCTCAGTTAGCGCTCAAC
TCAATGCCCCTCACTGGCGAGGCGAGGGCAAGGATGGAGGGGCAGCATCGCC
TGAGTTGGAGCAAAGCGGCCCGGCCGCCATGGGAGCAGCGAACCAACGGAGG
GATGCCGTGCTTTGTCGTGGCTGCTGTGGCCAATCCGGGCCCTTGGTTGGCTC
ACAGAGCGTTGCTGTGAGACCATGAGCTATTATTGCTAGGTACAGTATAGAGAG
AGGAGAGAGAGAGAGAGAGAGAGAGAGGGGAAAAAGGTGAGGTTGAAGTGA
GAAAAAAAAAAAAAAAAAAAAATCCAACCACTGACGGCTGCCGGCTCTGCCACC
CCCCTCCCTCCACCCCAGACCACCTGCACACTCAGCGCGCAGCATCACCTAAT
CTTGGCTCGCCTTCCCGCAGCTCAGGTTGTTTTTTTTTCTCTCTCCCTCGTCGA
AGCCGCCCTTGTTCCCTTATTTATTTCCCTCTCCATCCTTGTCTGCCTTTGGTCC
ATCTGCCCCTTTGTCTGCATCTCTTTTGCACGCATCGCCTTATCGTCGTCTCTTT
TTTCACTCACGGGAGCTTGACGAAGACCTGACTCGTGAGCCTCACCTGCTGATT
TCTCTCCCCCCCTCCCGACCGGCTTGACTTTTGTTTCTCCTCCAGTACCTTATCG
CGAAGCCGGAAGAACCTCTTAACCTCTAGATGAAAAAGCCTGAACTCACCGCCA
CGTCTGTCGAGAAGTTCCTGATCGAAAAGTTCGACAGCGTCTCCGACCTGATGC
AGCTCTCGGAGGGCGAAGAATCTCGTGCTTTCAGCTTCGATGTAGGAGGGCGT
GGATATGTCCTGCGGGTAAATAGCTGCGCCGATGGTTTCTACAAAGATCGTTAT
GTTTATCGGCACTTTGCATCGGCCGCGCTCCCGATTCCGGAAGTGCTTGACATT
GGGGAATTCAGCGAGAGCCTGACCTATTGCATCTCCCGCCGTGCACAGGGTGT
CACGTTGCAAGACCTGCCTGAAACCGAACTGCCCGCTGTTCTGCAGCCGGTCG
CGGAGGCCATGGATGCGATCGCTGCGGCCGATCTCAGCCAGACGAGCGGGTT
CGGCCCATTCGGACCGCAAGGAATCGGTCAATACACTACATGGCGTGATTTCAT
ATGCGCGATTGCTGATCCCCATGTGTATCACTGGCAAACTGTGATGGACGACAC
CGTCAGTGCGTCCGTCGCGCAGGCTCTCGATGAGCTGATGCTTTGGGCCGAGG
ACTGCCCCGAAGTCCGGCACCTCGTGCACGCGGATTTCGGCTCCAACAATGTC
CTGACGGACAATGGCCGCATAACAGCGGTCATTGACTGGAGCGAGGCGATGTT
CGGGGATTCCCAATACGAGGTCGCCAACATCTTCTTCTGGAGGCCGTGGTTGG

CTTGTATGGAGCAGCAGACGCGCTACTTCGAGCGGAGGCACCCGGAGCTTGCA
GGATCGCCGCGGCTCCGGGCGTATATGCTCCGCATTGGTCTTGACCAACTCTA
TCAGAGCTTGGTTGACGGCAATTTCGATGATGCAGCTTGGGCGCAGGGTCGAT
GCGACGCAATCGTCCGATCCGGAGCCGGGACTGTCGGGCGTACACAAATCGC
CCGCAGAAGCGCGGCCGTCTGGACCGATGGCTGTGTAGAAGTACTCGCCGATA
GTGGAAACCGACGCCCCAGCACTCGTCCGAGGGCAAAGGAATAGATGCATGGC
TTTCGTGACCGGGCTTCAAACAATGATGTGCGATGGTGTGGTTCCCGGTTGGC
GGAGTCTTTGTCTACTTTGGTTGTCTGTCGCAGGTCGGTAGACCGCAAATGAGC
AACTGATGGATTGTTGCCAGCGATACTATAATTCACATGGATGGTCTTTGTCGAT
CAGTAGCTAGTGAGAGAGAGAACATCTATCCACAATGTCGAGTGTCTATTAG
ACATACTCCGAGAATAAAGTCAACTGTGTCTGTGATCTAAAGATCGATTCGGCA
GTCGAGTAGCGTATAACAACTCCGAGTACCAGCGAAAGCACGTCGTGACAGGA
GCAGGGCTTTGCCAACTGCGCAACCTTGCTTGAATGAGGATACACGGGGTGCA
ACATGGCTGTACTGATCCATCGCAACCAAAATTTCTGTTTATAGATCAAGCTGGT
AGATTCCAATTACTCCACCTCTTGCGCTTCTCCATGACATGTAAGTGCACGTGGA
AACCATACCCAATATAACTTCGTATAATGTATGCTATACGAAGTTATAGGGCTCT
TGTCTGTT

SEQ ID NO: 3

LIC reception vector

TTTATCAGGGTTATTGTCTCATGAGCGGATACATATTTGAATGTATTTAGAAAAT
AAACAAATAGGGGTTCCGCGCACATTTCCCCGAAAAGTGCCACCTGACTAAACC
AGACAGACAGCTGTCTCTCCTCTCTAACATGTGAGCAAAAGGCCAGCAAAAGGC
CAGGAACCGTAAAAAGGCCGCGTTGCTGGCGTTTTTCCATAGGCTCCGCCCCC
CTGACGAGCATCACAAAAATCGACGCTCAAGTCAGAGGTGGCGAAACCCGACA
GGACTATAAAGATACCAGGCGTTTCCCCCTGGAAGCTCCCTCGTGCGCTCTCCT
GTTCCGACCCTGCCGCTTACCGGATACCTGTCCGCCTTTCTCCCTTCGGGAAGC
GTGGCGCTTTCTCATAGCTCACGCTGTAGGTATCTCAGTTCGGTGTAGGTCGTT
CGCTCCAAGCTGGGCTGTGTGCACGAACCCCCCGTTCAGCCCGACCGCTGCG
CCTTATCCGGTAACTATCGTCTTGAGTCCAACCCGGTAAGACACGACTTATCGC
CACTGGCAGCAGCCACTGGTAACAGGATTAGCAGAGCGAGGTATGTAGGCGGT
GCTACAGAGTTCTTGAAGTGGTGGCCTAACTACGGCTACACTAGAAGGACAGTA
TTTGGTATCTGCGCTCTGCTGAAGCCAGTTACCTTCGGAAAAAGAGTTGGTAGC
TCTTGATCCGGCAAACAAACCACCGCTGGTAGCGGTGGTTTTTTTGTTTGCAAG

CAGCAGATTACGCGCAGAAAAAAAGGATCTCAAGAAGATCCTTTGATCTTTTCTA
CGGGGTCTGACGCTCAGTGGAACGAAAACTCACGTTAAGGGATTTTGGTCATGA
GATTATCAAAAAGGATCTTCACCTAGATCCTTTTAAATTAAAAATGAAGTTTTAAA
TCAATCTAAAGTATATATGAGTAAACTTGGTCTGACAGTTACCAATGCTTAATCA
GTGAGGCACCTATCTCAGCGATCTGTCTATTTCGTTCATCCATAGTTGCCTGACT
CCCCGTCGTGTAGATAACTACGATACGGGAGGGCTTACCATCTGGCCCCAGTG
CTGCAATGATACCGCGAGACCCACGCTCACCGGCTCCAGATTTATCAGCAATAA
ACCAGCCAGCCGGAAGGGCCGAGCGCAGAAGTGGTCCTGCAACTTTATCCGCC
TCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGT
CGTTTGGTATGGCTTCATTCAGCTCCGGTTCCCAACGATCAAGGCGAGTTACAT
GATCCCCCATGTTGTGCAAAAAAGCGGTTAGCTCCTTCGGTCCTCCGATCGTTG
TCAGAAGTAAGTTGGCCGCAGTGTTATCACTCATGGTTATGGCAGCACTGCATA
ATTCTCTTACTGTCATGCCATCCGTAAGATGCTTTTCTGTGACTGGTGAGTACTC
AACCAAGTCATTCTGAGAATAGTGTATGCGGCGACCGAGTTGCTCTTGCCCGGC
GTCAATACGGGATAATACCGCGCCACATAGCAGAACTTTAAAAGTGCTCATCATT
GGAAAACGTTCTTCGGGGCGAAAACTCTCAAGGATCTTACCGCTGTTGAGATCC
AGTTCGATGTAACCCACTCGTGCACCCAACTGATCTTCAGCATCTTTTACTTTCA
CCAGCGTTTCTGGGTGAGCAAAAACAGGAAGGCAAAATGCCGCAAAAAAGGGA
ATAAGGGCGACACGGAAATGTTGAATACTCATACTCTTCCTTTTTCAATATTATTG
AAGCA

SEQ ID NO:4

pSEQ1flank5

CTAAATTGTAAGCGTTAATATTTTGTTAAAATTCGCGTTAAATTTTTGTTAAATCAG
CTCATTTTTTAACCAATAGGCCGAAATCGGCAAAATCCCTTATAAATCAAAAGAA
TAGACCGAGATAGGGTTGAGTGGCCGCTACAGGGCGCTCCCATTCGCCATTCA
GGCTGCGCAACTGTTGGGAAGGGCGTTTCGGTGCGGGCCTCTTCGCTATTACG
CCAGCTGGCGAAAGGGGGATGTGCTGCAAGGCGATTAAGTTGGGTAACGCCAG
GGTTTTCCCAGTCACGACGTTGTAAAACGACGGCCAGTGAGCGCGACGTAATA
CGACTCACTATAGGGCGAATTGGCGGAAGGCCGTCAAGGCCGCATGACTGAAT
AACTTCGGGCGGCCCCCCTGTCCAGCCTGACACGGTTCTGTTAGACTCGCTGG
CGGAGAAATGCATGGAGGTGCTCATGCAGGAGCACCATCTTCGGGTTTACTGC
GTTATGATCACAGCCCCGAACGCACTGCCGCGAGTGATCAAGAACGGAAGACG

GGAAATAGGGAACATGCTCTGCCGGCGCGAGTTTGACCTTGGCAACCTCCCAT
GCGTGCATGTCAAATTTGGCGTCGAACATGCGGTTCTCAACCTCCCGATTGGCG
TTGACCCCATTGGTGGTATCTGGTCACCAATCGCCTCGGACTCGAGAATCAATA
TCCTGGCTCCCGCCGATAAGCAGTATTCTGGAATCGACCGCAGAGAGGTTGTTA
TGGACGACCGGACGTCTACACCGCTCAACAATTTCAAGACCATCACCGATCTGA
TCCAGTGGCGTGTTGCTCGCCAGCCAGAGGAGCTCGCTTATTGTACCATTGAC
GGCAGGGGCAGAGAGGGCAAGGGGATTCCGTGGAAGAAGTTTGACTCCAAGG
TGGCGGCTGTGGCCATGTATCTGAAGAACAAAGTCAAGGTGCGGCCGGGCGAC
CACCTGGTCCTCATGTACACCCACTCCGAGGAGTTTGTCTTTGCCGTCCACGCG
GGAATCAACCTTGGCGCAGTCATTATTCCCATGGCGCCGCTTGACCAGAACCG
GCTCAACGAAGATGTCCCTGCTTTCCTGCACCTGATCGCTGACTACAAGGTTAA
GGCGGTCCTGGTCAACCAGGAAGTGGACCATTTGCTGAAGCTCAAGATCGTGT
CGAGCCACATCAAACAGTCCGCACAGATCCTGAAGATCTCGATGCCGAATACCT
ACAACACTTCGAAGCCACCTAAGCAGAACAACGGTCTTCGCGAGCTTGGGCTG
ACGATAGATCCCGCCTGGATCAGGCCTGGATACCCCGTCCTCATCTGGACATAC
TGGACGCCGGACCAACGGAGAATCGCCGTCCAGCTGGGGCATGATACCATCCT
GGGCATGTGCAAAGTGCAGAAGGAGACTTGTCAGATGACGAGCTTCCAGCCCG
TTCTCGGTTGCGTAAGAAGCACAACGGACTTGGTTTCGTGCACACGTGCCTGA
TGGGCATCTACGTTGGCACCGCCACCTACCTGCTGTCTCCTGTCGAGTTCGCC
CAAAATCCCATCTCTCTCTTTGTTACGTTGTCGAGGTACAAGATCAAGGACACCT
ATGCAACGCCGCAGATGCTTGACCATGCCATGTCGTCGATGCAGGCCAAGGGC
TTTACAATGCACGAACTGAAGAATATGATGATTACTGCAGAGGGCCGGCCGCGG
GTAGATGTATTCCAGAAGGTACGGATGCATTTTGCGAGCGCCGGGCTGGATAG
GACGGCCATCAACACGGTCTACTCGCATGTGCTCAACCCGATGATTGCTTCGAG
GTCTTACATGTGCATCGAGCCTATTGAGCTCTGGCTCGACACCAAGGCTCTTCG
ACGCGGCCTCGTCGTCCCGGTCGATCACGATTCAAACCCGCAAGCTCTTCTCCT
GCAGGATTCCGGCATGGTGCCGGTGTCTACCCAGATTGCCATTGTCAACCCCG
AGAGCCGCGCGCATTGCTACGATGGAGAATATGGCGAGATCTGGGTCGACTCC
GAGGCGTGCGTAAAGGCCTTTTACGGCTCCAAGGAAGCGTTTGACGTGGAGCG
CTTCGACGGCCGGACGGTCGACGGCGACCCCAACGTGCGATACGTGCGAACT
GGTGACTTGGGCTTTTTGTATAATGTCAACCGGCCTATCGGGCCCAACGGCGC
CCTGGTGGAGATGCAACTTGTTTGTGCTCGGTAGCATCGGCGAGACTTTTGAAA
TTAACGGTTTGAGTCACTTCCCCATGGATATTGAGCTGTCGGTGGAACGCTGCC
ACCGCAACATTGTACCCAACGGCTGGTAAGTACAGGGCCAACTCTTCTGTGAGA

TGCTACTTGACTAATAGTTGGTGATGTGCAGTGCTGTATTCCAAGCTGGTGGCT
TGGTCGTGGTCCTGGTAGAGGTGATAACAAGACACAGCCCGGCTCTTGTCTG
TTACTGGGCCTCATGGCCTTCCGCTCACTGCCCGCTTTCCAGTCGGGAAACCT
GTCGTGCCAGCTGCATTAACATGGTCATAGCTGTTTCCTTGCGTATTGGGCGCT
CTCCGCTTCCTCGCTCACTGACTCGCTGCGCTCGGTCGTTCGGGTAAAGCCTG
GGGTGCCTAATGAGCAAAAGGCCAGCAAAAGGCCAGGAACCGTAAAAAGGCCG
CGTTGCTGGCGTTTTTCCATAGGCTCCGCCCCCCTGACGAGCATCACAAAAATC
GACGCTCAAGTCAGAGGTGGCGAAACCCGACAGGACTATAAAGATACCAGGCG
TTTCCCCCTGGAAGCTCCCTCGTGCGCTCTCCTGTTCCGACCCTGCCGCTTACC
GGATACCTGTCCGCCTTTCTCCCTTCGGGAAGCGTGGCGCTTTCTCATAGCTCA
CGCTGTAGGTATCTCAGTTCGGTGTAGGTCGTTCGCTCCAAGCTGGGCTGTGT
GCACGAACCCCCCGTTCAGCCCGACCGCTGCGCCTTATCCGGTAACTATCGTC
TTGAGTCCAACCCGGTAAGACACGACTTATCGCCACTGGCAGCAGCCACTGGT
AACAGGATTAGCAGAGCGAGGTATGTAGGCGGTGCTACAGAGTTCTTGAAGTG
GTGGCCTAACTACGGCTACACTAGAAGAACAGTATTTGGTATCTGCGCTCTGCT
GAAGCCAGTTACCTTCGGAAAAAGAGTTGGTAGCTCTTGATCCGGCAAACAAAC
CACCGCTGGTAGCGGTGGTTTTTTTGTTTGCAAGCAGCAGATTACGCGCAGAAA
AAAAGGATCTCAAGAAGATCCTTTGATCTTTTCTACGGGGTCTGACGCTCAGTG
GAACGAAAACTCACGTTAAGGGATTTTGGTCATGAGATTATCAAAAAGGATCTTC
ACCTAGATCCTTTTAAATTAAAAATGAAGTTTTAAATCAATCTAAAGTATATATGA
GTAAACTTGGTCTGACAGTTACCAATGCTTAATCAGTGAGGCACCTATCTCAGC
GATCTGTCTATTTCGTTCATCCATAGTTGCCTGACTCCCCGTCGTGTAGATAACT
ACGATACGGGAGGGCTTACCATCTGGCCCCAGTGCTGCAATGATACCGCGAGA
ACCACGCTCACCGGCTCCAGATTTATCAGCAATAAACCAGCCAGCCGGAAGGG
CCGAGCGCAGAAGTGGTCCTGCAACTTTATCCGCCTCCATCCAGTCTATTAATT
GTTGCCGGGAAGCTAGAGTAAGTAGTTCGCCAGTTAATAGTTTGCGCAACGTTG
TTGCCATTGCTACAGGCATCGTGGTGTCACGCTCGTCGTTTGGTATGGCTTCAT
TCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCG
CAGTGTTATCACTCATGGTTATGGCAGCACTGCATAATTCTCTTACTGTCATGCC
ATCCGTAAGATGCTTTTCTGTGACTGGTGAGTACTCAACCAAGTCATTCTGAGAA
TAGTGTATGCGGCGACCGAGTTGCTCTTGCCCGGCGTCAATACGGGATAATAC
CGCGCCACATAGCAGAACTTTAAAAGTGCTCATCATTGGAAAACGTTCTTCGGG
GCGAAAACTCTCAAGGATCTTACCGCTGTTGAGATCCAGTTCGATGTAACCCAC

TCGTGCACCCAACTGATCTTCAGCATCTTTTACTTTCACCAGCGTTTCTGGGTGA
GCAAAAACAGGAAGGCAAAATGCCGCAAAAAAGGGAATAAGGGCGACACGGAA
ATGTTGAATACTCATACTCTTCCTTTTTCAATATTATTGAAGCATTTATCAGGGTT
ATTGTCTCATGAGCGGATACATATTTGAATGTATTTAGAAAAATAAACAAATAGG
GGTTCCGCGCACATTTCCCCGAAAAGTGCCAC

## Claims

1. Process for production of a technical enzyme composition, comprising the following steps:
(a) providing a fermentation medium with a glucose content of from 5 to 450 g/L;
(b) addition of at least one filamentous fungus cell wherein SEQ ID NO:1 has been disrupted;
(c) mixing of the fermentation medium and the at least one filamentous fungus cell for a time period of from 1 minute to 10 days at a temperature of from 20 to 35 °C;
(d) obtaining a technical enzyme composition.

2. Process according to claim 1, wherein the pH of the fermentation medium according to step (a) has been adjusted to a pH selected from pH 2.0 to 6.0.

3. Process according to any of the foregoing claims, wherein the fermentation medium further contains xylose and wherein the glucose to xylose ratio is selected from the range of from 1 to 3.5.

4. Process according to any of claims 1 or 2 wherein the fermentation medium further contains lactose and wherein the glucose to lactose ratio is selected from the range of from 1 to 10.

5. Process according to any of the foregoing claims, wherein no gluco-oligosaccharides have been added to the fermentation medium.

6. Process according to any of the foregoing claims, wherein no sophorose has been added to the fermentation medium.

7. Process according to any of the foregoing claims, further comprising step (ai) sterilization of the fermentation medium according to step (a).

8. Process according to any of the foregoing claims, wherein the fermentation medium has a potassium hydrogen phosphate content of from 0.5 to 10.0 g/L, a magnesium sulfate heptahydrate content of from 0.05 to 1 g/L, a calcium chloride dihydrate content of from 0.1 to 1 g/L, an ammonium sulfate content of from 1.5 to 4.5 g/L, an iron (II) sulfate heptahydrate content of from 0.005 to 0.1 g/L, a manganese sulfate content of from 0.00001 to 0.001 g/L, a zinc sulfate heptahydrate content of from 0.001 to 0.01 g/L and/or a copper sulfate pentahydrate content of from 0.0001 to 0.001.

9. Process according to any of the foregoing claims wherein the fermentation medium has a nitrogen content of from 0.05 to 2.0 g/L.

10. Process according to any of the foregoing claims, wherein the filamentous fungus cell is selected from the group consisting of *Acremonium, Aspergillus, Chaetomium, Emericella, Fusarium, Humicola, Hypocrea, Irpex, Magnaporte, Myceliophthora, Neurospora, Penicillium, Rhizopus, Talaromyces, Trichoderma* and *Trametes.*

11. Process according to any of the foregoing claims, wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidoreductase encoding sequence, at least one heterologous protease enzyme encoding sequence, at least one heterologous isomerase enzyme encoding sequence and/or at least one heterologous lytic polysaccharide monooxygenase enzyme encoding sequence.

12. Process according to any of the foregoing claims further comprising the step
(e) subjecting the technical enzyme composition according to step d) to a purification method.

13. Filamentous fungus cell wherein SEQ ID NO:1 has been disrupted.

14. Filamentous fungus cell according to claim 13, wherein SEQ ID NO:1 has been disrupted by deletion, mutation, modification of a promotor or any other regulatory sequence, generation of a stop codon or RNA interference.

15. Filamentous fungus cell according to any of claims 13 or 14, wherein the at least one filamentous fungus cell is a genetically modified filamentous fungus cell wherein the filamentous fungus cell comprises at least one heterologous beta-glucosidase enzyme encoding sequence, at least one heterologous cellulase enzyme encoding sequence, at least one heterologous xylanase enzyme encoding sequence, at least one heterologous beta-xylosidase enzyme encoding sequence, at least one heterologous pectinase enzyme encoding sequence, at least one heterologous oxidoreductase encoding sequence, at least one heterologous protease enzyme encoding sequence, at least one heterologous isomerase enzyme encoding sequence and/or at least one heterologous lytic polysaccharide monooxygenase enzyme encoding sequence.

16. Technical enzyme composition produced according to a process as defined in any of claims 1 to 12.

17. Use of a filamentous fungus cell as defined in any of claims 13 to 15 for the production of a technical enzyme composition.
